# EUROPEAN PATENT APPLICATION

(11) **EP 2 169 067 A1**
(43) Date of publication of application: **31.03.2010**
(21) Application number: 09160497.5
(22) Date of filing: 22.12.2005
(51) Int. Cl.: C12N 15/67, C12P 21/02, C07K 14/765, A61K 38/38, A61K 9/16, C12R 1/645, C12R 1/66, C12R 1/685, C12R 1/69

(54) **Recombinant production of serum albumin**

(30) Priority: 22.12.2004 DK 200401974; 01.03.2005 DK 200500310
(62) Divisional of application: 05823005.3
(71) Applicant: Novozymes A/S, 2880 Bagsvaerd (DK)
(72) Inventor: Christensen, Bjarke, 2800 Kgs. Lyngby (DK); Hjort, Carsten M., 3500 Vaerloese (DK); Poulsen, Thomas Agersten, 2750 Ballerup (DK); Bach, Poul, 3460 Birkeroed (DK)

(57) **Abstract**

The present invention relates to modified nucleotide sequences encoding serum albumin, wherein the mRNA sequence has been codon optimized for expression in a filamentous host cell. Furthermore the present invention relates to serum albumin produced in filamentous fungi or loaded by contacting serum albumin with an extract of a filamentous fungi culture. In another aspect the present invention relates to the use of said serum albumin in serum free cell culture media and also to a method of drying and agglomerating serum albumin thereby improving wettability and dispersability

## Description

### Field of the invention

The present invention relates to methods for recombinant expression of serum albumin in a filamentous fungal host organism, to modified nucleic acid sequences encoding bovine and human serum albumin proteins (BSA and HSA, respectively), to a loaded serum albumin product, to a use of the loaded serum albumin, and to a process for the preparation of a dry particle as well as a dry particle comprising serum albumin.

### Background of the invention

Serum albumin, especially bovine serum albumin (BSA), is one of the most widely used proteins in the field of molecular biology and industry.

Serum albumin like e.g. BSA or HSA has previously been obtained by blood fractionation. This way of providing serum albumin suffers from the drawback of possible contamination with pathogens. In the case of BSA, the presence of prions thought to be responsible for mad-cow disease (BSE) is in particular a problem associated with the production of BSA by blood fractionation. For HSA there is a risk of possible contamination by hepatitis and immunodeficiency viruses like HIV, when HSA is produced by blood fractionation.
Alternative methods for providing serum albumin which is free of contaminants and which can be produced in economical yields are therefore desirable.

Recombinant expression of BSA/HSA would solve the problem of contamination described above. Also such recombinant serum albumin would be better defined in terms of other contaminants originating from blood plasma like e.g. blood plasma proteins and peptides (globulins). For many therapeutic uses and for obtaining more defined cell culture media, especially serum free media, recombinant serum albumin would be preferable.

Recombinant expression of proteins is however not always straight forward and obtaining sufficient yields is hard to predict for a given protein in a given expression host organism. The recombinant expression of albumin in yeast has been reported in WO 00/44772, EP 0683233 A2, and US 5,612,196.

After expression in a host organism or purified from serum, albumin can be processed in order to improve further downstream applications. It is well known in the art to spray dry serum albumin (SA) to obtain an agglomerate free powder. WO 04/058156 discloses methods providing stable powder particles containing bioactive materials. The methods include, e.g., high pressure spraying of the bioactive materials in solution or suspension, with viscosity enhancing agents and/or surfactants.

WO 03/087335 discloses methods and compositions to preserve bioactive materials. The methods include high-pressure gas spraying and/or near supercritical spraying of formulations followed by drying in a stream of conditioned gas to form stable powder particles containing bioactive materials.

Serum albumin (SA) is often used in applications were it is of great importance that the SA is sterile. The problem has been how to obtain a sterile SA product and keeping the SA sterile upon storage and transport. A known method to use is freeze drying, however, there are some drawbacks in using freeze drying. During freeze drying some SA monomers reacts into dimers and trimers. This starting polymerization is fundamentally changing the properties of SA, in a way that makes the SA unusable for desired applications. During simple spray drying primary particles with a very low particle size are obtained. Said primary particles can not readily disperse in liquids. The primary particles stick together and make a gel like substance when added to a liquid. It would be desirable to obtain a SA product which readily disperses when added to a liquid and is thus fast dissolving.

The present invention provides a method for the efficient recombinant expression of serum albumin, in a filamentous fungal host suitable for industrial production. Further more the present invention provides a serum albumin product which can replace albumin produced from serum in all previously described uses and which in addition has several advantageous properties when used in serum free cell culture medium. In an additional aspect the present invention provides a method for improving the solubility of albumin.

### Summary of the invention

A first aspect of the present invention relates to a method for recombinant expression of a wild type serum albumin polypeptide in a filamentous fungal host organism comprising expressing a modified nucleic acid sequence encoding a wild type serum albumin polypeptide in a filamentous fungal host organism, wherein the modified nucleic acid sequence differs in at least one codon from each wild type nucleic acid sequence encoding said wild type serum albumin polypeptide.

In a second aspect the present invention relates to a method for recombinant expression of wild type serum albumin in a filamentous fungal host organism, comprising the steps:
i) providing a nucleic acid sequence encoding wild type serum albumin said nucleic acid sequence comprising at least one modified codon, wherein the modification does not change the amino acid encoded by said codon and the nucleic acid sequence of said codon is different compared to the corresponding codon in the nucleic acid sequence encoding the wild type gene;
ii) expressing the modified nucleic acid sequence in the filamentous fungal host.

A third aspect of the present invention relates to a modified nucleic acid sequence encoding a wild type bovine serum albumin polypeptide and capable of expression in a filamentous fungal host organism, wherein said modified nucleic acid sequence differs in at least one codon from each wild type nucleic acid sequence encoding said wild type bovine serum albumin polypeptide.

A fourth aspect of the present invention relates to a modified nucleic acid sequence encoding a wild type human serum albumin polypeptide and capable of expression in a filamentous fungal host organism, wherein said modified nucleic acid sequence differs in at least one codon from each wild type nucleic acid sequence encoding said wild type human serum albumin polypeptide.

A fifth aspect of the present invention relates to a modified nucleic acid sequence encoding the wild type BSA protein and capable of expression in a filamentous fungal host organism, which modified nucleic acid sequence is obtainable by:
i) providing the wild type nucleic acid sequence encoding BSA;
ii) modifying at least one codon, wherein the modification does not change the amino acid encoded by said codon and the nucleic acid sequence of said codon is different compared to the corresponding codon in the wild type gene.

A sixth aspect of the present invention relates to a modified nucleic acid sequence encoding the wild type HSA protein and capable of expression in a filamentous fungal host organism, which modified nucleic acid sequence is obtainable by:
i) providing the wild type nucleic acid sequence encoding HSA;
ii) modifying at least one codon, wherein the modification does not change the amino acid encoded by said codon and the nucleic acid sequence of said codon is different compared to the corresponding codon in the wild type gene.

In a seventh aspect the present invention relates to a modified nucleic acid sequence encoding the wild type BSA protein and capable of expression in a filamentous fungal host organism, wherein:
a) the modified sequence has at least 77% identity with SEQ ID NO: 5; or
b) the modified sequence hybridizes under high stringency conditions with a polynucleotide probe consisting of the complementary strand of nucleotides 1 to 1821 of SEQ ID NO: 5.

In an eight aspect the present invention relates to a modified nucleic acid sequence encoding the wild type HSA protein and capable of expression in a filamentous fungal host organism, wherein:
a) the modified sequence has at least 77% identity with SEQ ID NO: 7; or
b) the modified sequence hybridizes under high stringency conditions with a polynucleotide probe consisting of the complementary strand of nucleotides 1 to 1827 of SEQ ID NO: 7.

In a ninth aspect the present invention relates to a loaded serum albumin obtainable by:
i) recombinant expression of a nucleic acid sequence encoding the serum albumin in a filamentous fungal host cell; and/or
ii) loading the serum albumin by contacting said serum albumin with a cell extract derived from filamentous fungal cells.

A tenth aspect of the invention relates to a use of the loaded serum albumin according to the invention, in a cell culture medium.

In a further aspect the invention relates to a dry particle comprising serum albumin, wherein said particle is dried and agglomerated.

Furthermore the present invention relates to a method of producing a dried and agglomerated product comprising serum albumin, the method comprising the steps of:
a) drying a liquid composition comprising serum albumin; and
b) agglomerating the dried product.

In another aspect the present invention relates to a product comprising serum albumin, wherein said product is dried and have a particle size of at least 50 microns.

### Brief description of the drawings

The invention is further illustrated by the accompanying drawings in which:

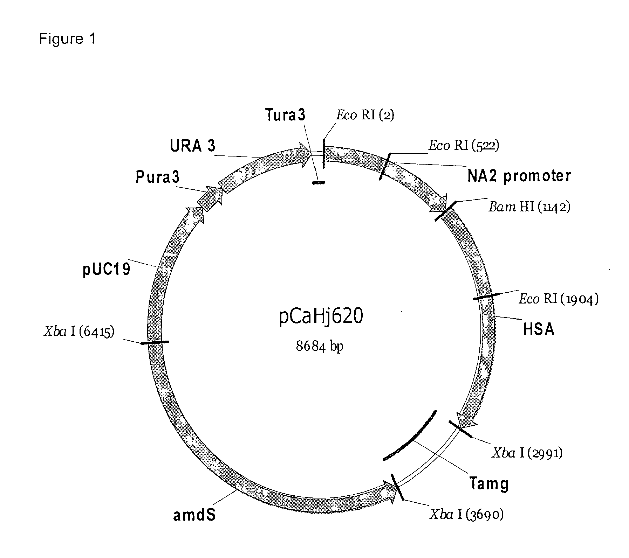

Figure 1 shows a restriction map of the Aspergillus expression plasmid pCaHj620 comprising a synthetic HSA gene according to the invention. The NA2 promoter is the modified neutral amylase promoter from Aspergillus niger. Tamg is the amyloglycosidase terminator from Aspergillus niger. HSA is the synthetic human serum albumin gene. amdS is the acetamidase gene from Aspergillus nidulans. pUC 19 is a fragment of the Escherichia coli vector pUC19. Pura3, URA3 and Tura3 are the promoter region ORF and terminator sequence of the Saccharomyces cerevisiae URA3 gene.

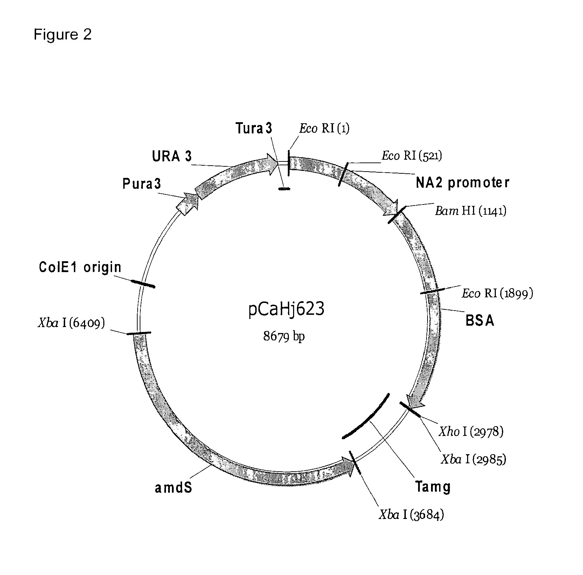

Figure 2 shows a restriction map of the Aspergillus expression plasmid pCaHj623 comprising a synthetic BSA gene according to the invention. NA2 promoter is the modified neutral amylase promoter from Aspergillus niger. Tamg is the amyloglycosidase terminator from Aspergillus niger. BSA is the synthetic bovine serum albumin gene. amdS is the acetamidase gene from Aspergillus nidulans. pUC 19 is a fragment of the Escherichia coli vector pUC19. Pura3, URA3 and Tura3 are the promoter region ORF and terminator sequence of the Saccharomyces cerevisiae URA3 gene.

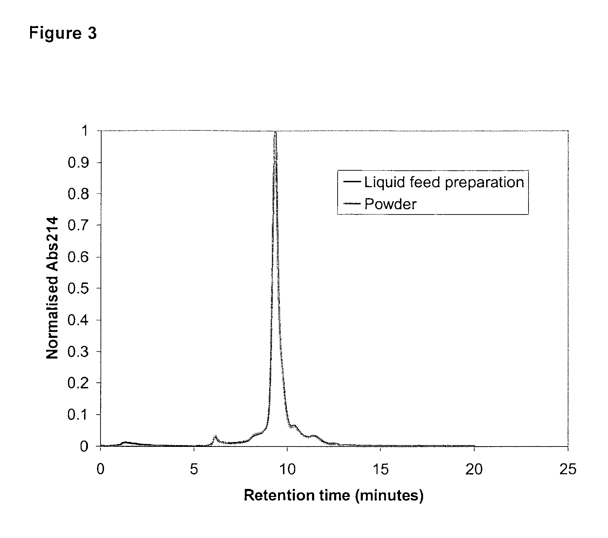

Figure 3 shows the HPLC analysis of the spray dried powder vs. the r-HSA concentrates used. The results show that no significant polymerisation takes place during spray drying as the elution profiles are essential identical.

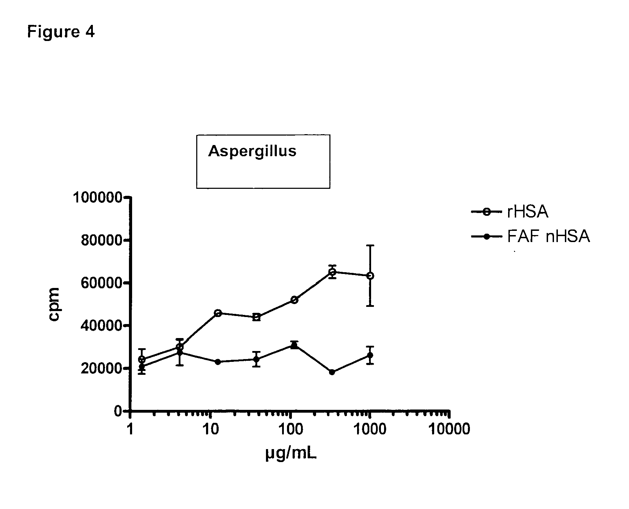

Figure 4 shows the effect of replacing natural HSA purified from serum in cell culture medium with rHSA expressed in *A. oryzae.* The figure shows cell growth, measured by incorporation of radioactively labelled thymidine, as a function of the concentration of HSA in the culture medium in µg/ml.

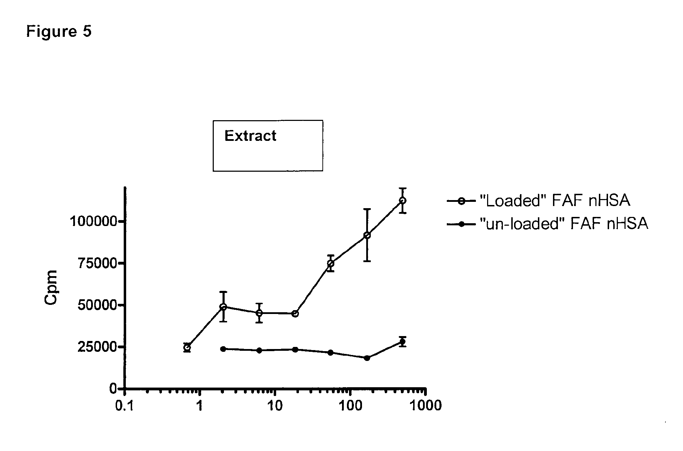

Figure 5 shows the effect of replacing natural HSA purified from serum in cell culture medium with loaded HSA, wherein the loaded HSA was obtained by incubating a fatty acid free commercial HSA product with a cell lysate derived from a culture of *A. oryzae.* The figure shows cell growth, measured by incorporation of radioactively labelled thymidine, as a function of the concentration of HSA in the culture medium in µg/ml.

### Detailed description of the invention

### Serum albumin

Serum albumin according to the present invention comprises a class of small globular proteins found in blood, synovial fluid, milk and other mammalian secretions. Serum albumin is the protein of the highest concentration in plasma, and transports many small molecules in the blood (e.g. bilirubin, calcium, progesterone and drugs). Serum albumin belongs to the ALB/AFP/VDB family.

In the context of the present invention the term "wild type serum albumin polypeptide" is to be understood as a naturally occurring mature serum albumin polypeptide. For most genes different alleles of the same gene exist naturally, i.e. different sequences of a gene that occupy a given genetic locus on a chromosome exist naturally. Some of these differences are only present at the nucleic acid level while others are also present at the amino acid level. In the context of the present invention the term "wild type serum albumin polypeptide" is intended to encompass all the naturally occurring mature serum albumin polypeptides. For example "wild type human serum albumin polypeptide" and "wild type bovine serum albumin polypeptide" encompass all naturally occurring mature polypeptides obtainable from human and bovine, respectively. In the context of the present invention the term "mature" is to be understood as the mature part of the translated amino acid sequence. For some polypeptides the amino acid sequence which is translated from the mRNA includes besides the mature polypeptide also a signal peptide and/or a pro-peptide. Thus the signal peptide and the pro-peptide are not regarded as being part of the mature peptide.

In a particular embodiment of the present invention the serum albumin may be human or bovine serum albumin, i.e. HSA or BSA. Several variations in the amino acid sequence of the naturally occurring BSA or HSA proteins have been reported in the literature (Meloun et al., FEBS Lett. 58:134-137 (1975); Lawn et al., Nucleic Acid Res. 9:6103-6114 (1981); Dugaiczyk et al., Proc. Natl. Acad. Sci. U.S.A. 79:71-75 (1982)).

In the context of the present invention a wild type HSA polypeptide is to be understood as to include any of the mature polypeptides described in Swissprot entry P02768, and a wild type BSA polypeptide is to be understood as to include any of the mature polypeptides described in Swissprot entry P02769.

In particular the wild type BSA polypeptide may be the mature peptide shown in SEQ ID NO: 1 or 2, i.e. amino acids 1-583 of SEQ ID NO: 1 or SEQ ID NO: 2.

In particular the wild type HSA polypeptide may be the mature peptide shown in SEQ ID NO: 3 or 4, i.e. amino acids 1-585 of SEQ ID NO: 3 or SEQ ID NO: 4.

The term "wild type nucleic acid sequence encoding a wild type serum albumin polypeptide" is in the context of the present invention to be understood as a naturally occurring nucleic acid sequence encoding the translated sequence of a wild type serum albumin polypeptide, i.e. besides the nucleic acid sequence encoding the mature polypeptide it also includes the nucleic acid sequence encoding e.g. the signal peptide and the pro-peptide. The translated sequence is also known as CDS. As described above different alleles of a particular gene often exist and "wild type nucleic acid sequence encoding a wild type serum albumin polypeptide" is in the context of the present invention intended to encompass all such naturally occurring nucleic acid sequences encoding a particular wild type serum albumin polypeptide.

In the context of the present invention a wild type nucleic acid sequence encoding a wild type human serum albumin polypeptide is to be understood as to include any of the CDS sequences described in the references which are included in Swissprot entry P02768, and a wild type nucleic acid sequence encoding a wild type bovine serum albumin polypeptide is to be understood as to include any of the CDS sequences described in the references which are included in Swissprot entry P02769.

In particular the wild type nucleic acid sequence encoding a wild type bovine serum albumin polypeptide may be the CDS shown in SEQ ID NO: 1, i.e. nucleotide 1-1821 of SEQ ID NO: 1.

In particular the wild type nucleic acid sequence encoding a wild type human serum albumin polypeptide may be the CDS shown in SEQ ID NO: 3, i.e. nucleotides 1-1827 of SEQ ID NO: 3.

### Method of the invention

Bovine serum albumin (BSA) is a widely used protein within the field of molecular biology and therefore a commercially interesting product. Human serum albumin (HSA) is especially used in therapeutic applications but can also replace BSA in most applications were BSA is commonly used.

The known way of producing BSA or HSA as described above involves blood fractionation which in turn may lead to possible contamination with pathogens.

According to the present invention the BSA or HSA is produced as a recombinant protein in a filamentous fungal host cell, which solves the problem of contamination as well as observed problems of expressing the wild type genes in filamentous fungi.

Recombinant expression of proteins is not always straight forward and it is hard to predict whether the desired product can in fact be produced in a particular production host organism and whether product yields will be sufficient for establishing an economical production.

In respect of BSA and HSA a first attempted was made to express the proteins from a normal wild type nucleic acid sequences (the nucleic acid sequences shown in SEQ ID NO: 1 and 3, respectively) in a filamentous fungal. However, no expression could be detected.

Several possibilities or combination of possibilities exist for explaining the lack of expression observed in filamentous fungal hosts. In general expression of a secreted and correctly processed albumin in a filamentous fungus involves a number of steps any of which could be a limiting step.

First the inserted albumin gene is transcribed to hnRNA. Then the hnRNA is transported from the nucleus to the cytosol, and during this process it is maturated to mRNA. Generally a mRNA pool is established in the cytosol in order to sustain translation. The mRNA is then translated to a protein precursor, and this precursor is subsequently secreted to the endoplasmatic reticulum (ER) either co-translationally or post-translationally. Upon translocation into the ER the secretion signal peptide is cleaved of by a signal peptidase, and the resulting protein is folded in the ER. Secretion of the protein to the golgi apparatus follows when proper folding has been recognized by the cell. Here the propeptide will be cleaved to release the mature albumin. Thus numerous possibilities exist for preventing sufficient expression of a gene sequence in a given host organism.

In order to provide efficient expression of a polynucleotide sequence encoding a desired protein the translation process has to be efficient. One object of the present invention is therefore to optimize the mRNA sequence encoding the serum albumin protein in order to obtain sufficient expression in a filamentous fungal host cell.

In one embodiment the present invention relates to a method for recombinant expression of a wild type serum albumin polypeptide in a filamentous fungal host organism comprising expressing a modified nucleic acid sequence encoding a wild type serum albumin polypeptide in a filamentous fungal host organism, wherein the modified nucleic acid sequence differs in at least one codon from each wild type nucleic acid sequence encoding said wild type serum albumin polypeptide.

The modified nucleic acid sequence may be obtained by a) providing a wild type nucleic acid sequence encoding a wild type serum albumin polypeptide and b) modifying at least one codon of said nucleic acid sequence so that the modified nucleic acid sequence differs in at least one codon from each wild type nucleic acid sequence encoding said wild type serum albumin polypeptide. Methods for modifying nucleic acid sequences are well known to a person skilled in the art. In a particular embodiment said modification does not change the identity of the amino acid encoded by said codon.

Thus in another aspect the object of the present invention is provided by a method for recombinant expression of wild type serum albumin in a filamentous fungal host organism, comprising the steps:
i) providing a nucleic acid sequence encoding wild type serum albumin said nucleic acid sequence comprising at least one modified codon, wherein the modification does not change the amino acid encoded by said codon and the nucleic acid sequence of said codon is different compared to the corresponding codon in the nucleic acid sequence encoding the wild type gene;
ii) expressing the modified nucleic acid sequence in the filamentous fungal host.

The starting nucleic acid sequence to be modified according to this embodiment is a wild type nucleic acid sequence encoding the serum albumin of interest.

Modifications according to the invention, comprises any modification of the base triplet and in a particular embodiment they comprise any modification which does not change the identity of the amino acid encoded by said codon, i.e. the amino acid encoded by the original codon and the modified codon is the same. In most cases the modification will be at the third position, however, in a few cases the modification may also be at the first or the second position. How to modify a codon also without modifying the resulting amino acid is known to the skilled person.

For both of the above embodiments the number of codon which should differ or the number of modifications needed in order to obtain sufficient expression may vary. Thus according to a further embodiment of the invention the modified nucleic acid sequence differs in at least 2 codons from each wild type nucleic acid sequence encoding said wild type serum albumin polypeptide or at least 2 codons have been modified, particularly at least 3 codons, more particularly at least 5 codons, more particularly at least 10 codons, more particularly at least 15 codons, even more particularly at least 25 codons.

It has furthermore been found, that by changing the codon usage of the wild type nucleic acid sequence to be selected among the codons preferably used by the filamentous fungus used as a host, the expression of BSA and HSA is now possible. Such codons are said to be "optimized" for expression.

In one attempt to express BSA and HSA as recombinant proteins expressed from the wild type sequence in fungal systems the level of expression was very low, about 1 mg/l using the native genes and *Aspergillus* as the host cell.

Due to the degeneracy of the genetic code and the preference of certain preferred codons in particular organisms/cells the expression level of a protein in a given host cell can in some instances be improved by optimizing the codon usage. In the present case the yields of BSA and HSA were increased dramatic when wild type nucleic acid sequences encoding BSA and HSA were optimized by, among other things, codon optimization and expressed in *Aspergillus.*

In the present invention "codon optimized" means that due to the degeneracy of the genetic code more than one triplet codon can be used for each amino acid. Some codons will be preferred in a particular organism and by changing the codon usage in a wild type gene to a codon usage preferred in a particular expression host organism the codons are said to be optimized. Codon optimization can be performed e.g. as described in Gustafsson et al., 2004, (Trends in Biotechnology vol. 22 (7); Codon bias and heterologous protein expression), and US 6,818,752.

Codon optimization may be based on the average codon usage for the host organism or it can be based on the codon usage for a particular gene which is know to be expressed in high amounts in a particular host cell.

In one embodiment of the invention the serum albumin protein is encoded by a modified nucleic acid sequence codon optimized in at least 10 % of the codons, more particularly at least 20%, or at least 30 %, or at least 40 %, or particularly at least 50 %, more particularly at least 60 %, and more particularly at least 75%. Thus the modified nucleic acid sequence may differ in at least 10 % of the codons from each wild type nucleic acid sequence encoding said wild type serum albumin polypeptide, more particularly in at least 20%, or in at least 30 %, or in at least 40 %, or particularly in at least 50 %, more particularly in at least 60 %, and more particularly in at least 75%. In particular said codons may differ because they have been codon optimized as compared with a wild type nucleic acid sequence encoding a wild type serum albumin polypeptide.

Particularly 100% of the nucleic acid sequence has been codon optimized to match the preferred codons used in filamentous fungi.

In a particular embodiment the codon optimization is based on the codon usage of alpha amylase from Aspergillus oryzae, also known as Fungamyl^{™} (PCT/DK 2004/000558; SEQ ID NO: 2), which is a protein known to be expressed in high levels in filamentous fungi. In the present context an expression level corresponding to at least 20 % of the total amount of secreted protein constitutes the protein of interest is considered a high level of expression. Particularly at least 30 %, more particularly at least 40 %, even more particularly at least 50 %.

In a particular embodiment, the modified nucleic acid sequence encoding BSA or HSA is selected from the group consisting of SEQ ID NO: 5 (BSA) and SEQ ID NO: 7 (HSA).

In practice the optimization according to the invention comprises the steps:
i) the nucleic acid sequence encoding the serum albumin is codon optimized as explained in more detail below;
ii) check the resulting modified sequence for a balanced GC-content (approximately 45-55%); and
iii) check or edit the resulting modified sequence from step ii) as explained below.

### Codon optimization protocol:

The codon usage of a single gene, a number of genes or a whole genome can be calculated with the program cusp from the EMBOSS-package (http://www.rfcgr.mrc.ac.uk/Software/EMBOSS/).

The starting point for the optimization is the amino acid sequence of the protein or a nucleic acid sequence coding for the protein together with a codon-table. By a codon-optimized gene, we understand a nucleic acid sequence, encoding a given protein sequence and with the codon statistics given by a codon table.

The codon statistics referred to is a column in the codon-table called "Fract" in the output from cusp-program and which describes the fraction of a given codon among the other synonymous codons. We call this the local score. If for instance 80% of the codons coding for F is TTC and 20% of the codons coding for F are TTT, then the codon TTC has a local score of 0.8 and TTT has a local score of 0.2.

The codons in the codon table are re-ordered first encoding amino acid (e.g. alphabetically) and then increasingly by the score. In the example above, ordering the codons for F as TTT, TTC. Cumulated scores for the codons are then generated by adding the scores in order. In the example above TTT has a cumulated score of 0.2 and TTC has a cumulated score of 1. The most used codon will always have a cumulated score of 1.

In order to generate a codon optimized gene the following is performed. For each position in the amino acid sequence, a random number between 0 and 1 is generated. This is done by the random-number generator on the computer system on which the program runs. The first codon is chosen as the codon with a cumulated score greater than or equal to the generated random number. If, in the example above, a particular position in the gene is "F" and the random number generator gives 0.5, TTC is chosen as codon.

The strategy for avoiding introns is to make sure that there are no branch points. This was done by making sure that the consensus sequence for branch-point in Aspergillus oryzae: CT[AG]A[CT] was not present in the sequence. The inventors of the present invention have also found that the sequence [AG]CT[AG]A[AG] may be recognised as a branch points in introns. Thus in a particular embodiment of the present invention such sequences may also be modified or be removed according to a method of the present invention. This was done in a post processing step, where the sequence was scanned for the presence of this motif, and each occurrence was removed by changing codons in the motif to synonymous codons, choosing codons with the best local score first.

A codon table showing the codon usage of the alpha amylase from Aspergillus oryzae is given below.

**Table 1. Codon usage for the A. oryzae alpha amylase**

| (CUSP codon usage file) | | | | |
|---|---|---|---|---|
| Codon | Amino acid | Fract | /1000 | Number |
| GCA | A | 0.286 | 24.000 | 12 |
| GCC | A | 0.357 | 30.000 | 15 |
| GCG | A | 0.238 | 20.000 | 10 |
| GCT | A | 0.119 | 10.000 | 5 |
| TGC | C | 0.222 | 4.000 | 2 |
| TGT | C | 0.778 | 14.000 | 7 |
| GAC | D | 0.524 | 44.000 | 22 |
| GAT | D | 0.476 | 40.000 | 20 |
| GAA | E | 0.417 | 10.000 | 5 |
| GAG | E | 0.583 | 14.000 | 7 |
| TTC | F | 0.800 | 24.000 | 12 |
| TTT | F | 0.200 | 6.000 | 3 |
| GGA | G | 0.233 | 20.000 | 10 |
| GGC | G | 0.419 | 36.000 | 18 |
| GGG | G | 0.116 | 10.000 | 5 |
| GGT | G | 0.233 | 20.000 | 10 |
| CAC | H | 0.571 | 8.000 | 4 |
| CAT | H | 0.429 | 6.000 | 3 |
| ATA | I | 0.071 | 4.000 | 2 |
| ATC | I | 0.679 | 38.000 | 19 |
| ATT | I | 0.250 | 14.000 | 7 |
| AAA | K | 0.350 | 14.000 | 7 |
| AAG | K | 0.650 | 26.000 | 13 |
| CTA | L | 0.081 | 6.000 | 3 |
| CTC | L | 0.351 | 26.000 | 13 |
| CTG | L | 0.162 | 12.000 | 6 |
| CTT | L | 0.108 | 8.000 | 4 |
| TTA | L | 0.027 | 2.000 | 1 |
| TTG | L | 0.270 | 20.000 | 10 |
| ATG | M | 1.000 | 22.000 | 11 |
| AAC | N | 0.885 | 46.000 | 23 |
| AAT | N | 0.115 | 6.000 | 3 |
| CCA | P | 0.136 | 6.000 | 3 |
| CCC | P | 0.364 | 16.000 | 8 |
| CCG | P | 0.227 | 10.000 | 5 |
| CCT | P | 0.273 | 12.000 | 6 |
| CAA | Q | 0.250 | 10.000 | 5 |
| CAG | Q | 0.750 | 30.000 | 15 |
| AGA | R | 0.000 | 0.000 | 0 |
| AGG | R | 0.300 | 6.000 | 3 |
| CGA | R | 0.200 | 4.000 | 2 |
| CGC | R | 0.200 | 4.000 | 2 |
| CGG | R | 0.200 | 4.000 | 2 |
| CGT | R | 0.100 | 2.000 | 1 |
| AGC | S | 0.162 | 12.000 | 6 |
| AGT | S | 0.108 | 8.000 | 4 |
| TCA | S | 0.108 | 8.000 | 4 |
| TCC | S | 0.243 | 18.000 | 9 |
| TCG | S | 0.270 | 20.000 | 10 |
| TCT | S | 0.108 | 8.000 | 4 |
| ACA | T | 0.250 | 20.000 | 10 |
| ACC | T | 0.325 | 26.000 | 13 |
| ACG | T | 0.200 | 16.000 | 8 |
| ACT | T | 0.225 | 18.000 | 9 |
| GTA | V | 0.129 | 8.000 | 4 |
| GTC | V | 0.387 | 24.000 | 12 |
| GTG | V | 0.323 | 20.000 | 10 |
| GTT | V | 0.161 | 10.000 | 5 |
| TGG | W | 1.000 | 24.000 | 12 |
| TAC | Y | 0.686 | 48.000 | 24 |
| TAT | Y | 0.314 | 22.000 | 11 |
| TAA | * | 0.000 | 0.000 | 0 |
| TAG | * | 0.000 | 0.000 | 0 |
| TGA | * | 1.000 | 2.000 | 1 |

### Introns

Eukaryotic genes may be interrupted by intervening sequences (introns) which must be modified in precursor transcripts in order to produce functional mRNAs. This process of intron removal is known as pre-mRNA splicing. Usually, a branchpoint sequence of an intron is necessary for intron splicing through the formation of a lariat. Signals for splicing reside directly at the boundaries of the intron splice sites. The boundaries of intron splice sites usually have the consensus intron sequences GT and AG at their 5' and 3' extremities, respectively. While no 3' splice sites other than AG have been reported, there are reports of a few exceptions to the 5' GT splice site. For example, there are precedents where CT or GC is substituted for GT at the 5' boundary. There is also a strong preference for the nucleotide bases ANGT to follow GT where N is A, C, G, or T (primarily A or T in *Saccharomyces* species), but there is no marked preference for any particular nucleotides to precede the GT splice site. The 3' splice site AG is primarily preceded by a pyrimidine nucleotide base (Py), i.e., C or T.

The number of introns that can interrupt a fungal gene ranges from one to twelve or more introns (Rymond and Rosbash, 1992, In, E.W. Jones, J.R. Pringle, and J.R. Broach, editors, The Molecular and Cellular Biology of the Yeast Saccharomyces, pages 143-192, Cold Spring Harbor Laboratory Press, Plainview, New York; Gurr et al., 1987, In Kinghorn, J.R. (ed.), Gene Structure in Eukaryotic Microbes, pages 93-139, IRL Press, Oxford). They may be distributed throughout a gene or situated towards the 5' or 3' end of a gene. In *Saccharomyces cerevisiae*, introns are located primarily at the 5' end of the gene. Introns may be generally less than 1 kb in size, and usually are less than 400 bp in size in yeast and less than 100 bp in filamentous fungi.

The *Saccharomyces cerevisiae* intron branchpoint sequence 5'-TACTAAC-3' rarely appears exactly in filamentous fungal introns (Gurr *et al.,* 1987, *supra*). Sequence stretches closely or loosely resembling TACTAAC are seen at equivalent points in filamentous fungal introns with a general consensus NRCTRAC where N is A, C, G, or T, and R is A or G. For example, the fourth position T is invariant in both the *Neurospora* crassa and *Aspergillus nidulans* putative consensus sequences. Furthermore, nucleotides G, A, and C predominate in over 80% of the positions 3, 6, and 7, respectively, although position 7 in *Aspergillus nidulans* is more flexible with only 65% C. However, positions 1, 2, 5, and 8 are much less strict in both *Neurospora* crassa and *Aspergillus nidulans.* Other filamentous fungi have similar branchpoint stretches at equivalent positions in their introns, but the sampling is too small to discern any definite trends.

The heterologous expression of a gene encoding a polypeptide in a fungal host strain may result in the host strain incorrectly recognizing a region within the coding sequence of the gene as an intervening sequence or intron. For example, it has been found that intron-containing genes of filamentous fungi are incorrectly spliced in *Saccharomyces cerevisiae* (Gurr et al., 1987, In Kinghorn, J.R. (ed.), Gene Structure in Eukaryotic Microbes, pages 93-139, IRL Press, Oxford). Since the region is not recognized as an intron by the parent strain from which the gene was obtained, the intron is called a cryptic intron. This improper recognition of an intron, referred to herein as a cryptic intron, may lead to aberrant splicing of the precursor mRNA molecules resulting in no production of biologically active polypeptide or in the production of several populations of polypeptide products with varying biological activity.

"Cryptic intron" is defined herein as a region of a coding sequence that is incorrectly recognized as an intron which is excised from the primary mRNA transcript. A cryptic intron preferably has 10 to 1500 nucleotides, more preferably 20 to 1000 nucleotides, even more preferably 30 to 300 nucleotides, and most preferably 30 to 100 nucleotides.

The presence of cryptic introns can in particular be a problem when trying to express proteins in organisms which have a less strict requirement to what sequences are necessary in order to define an intron. Such "sloppy" recognition can result e.g. when trying to express recombinant proteins in fungal expression systems.

Cryptic introns can be identified by the use of Reverse Transcription Polymerase Chain Reaction (RT-PCR). In RT_PCR, mRNA is reverse transcribed into single stranded cDNA that can be PCR amplified to double stranded cDNA. PCR primers can then be designed to amplify parts of the single stranded or double stranded cDNA, and sequence analysis of the resulting PCR products compared to the sequence of the genomic DNA reveals the presence and exact location of cryptic introns (T. Kumazaki et al. (1999) J. Cell. Sci. 112, 1449 - 1453).

According to one embodiment of the invention the modification introduced into the wild type gene sequence will optimize the mRNA for expression in a particular host organism. In the present invention the host organism or host cell comprises a group of fungi referred to as filamentous fungi as explained in more detail below.

### Filamentous fungal host cell

The host cell of the invention is a filamentous fungus represented by the following groups of *Ascomycota*, include, *e.g., Neurospora, Eupenicillium (=Penicillium), Emericella* (*=Aspergillus*), *Eurotium* (*=Aspergillus*).

In a preferred embodiment, the filamentous fungus includes all filamentous forms of the subdivision *Eumycota* and *Oomycota* (as defined by Hawksworth et al., In, Ainsworth and Bisby's Dictionary of The Fungi, 8th edition, 1995, CAB International, University Press, Cambridge, UK). The filamentous fungi are characterized by a vegetative mycelium composed of chitin, cellulose, glucan, chitosan, mannan, and other complex polysaccharides. Vegetative growth is by hyphal elongation and carbon catabolism is obligately aerobic.

In a more preferred embodiment, the filamentous fungal host cell is a cell of a species of, but not limited to, *Acremonium, Aspergillus, Fusarium, Humicola, Mucor, Myceliophthora, Neurospora, Penicillium, Thielavia, Tolypocladium*, and *Trichoderma* or a teleomorph or synonym thereof. In an even more preferred embodiment, the filamentous fungal host cell is an *Aspergillus* cell. In another even more preferred embodiment, the filamentous fungal host cell is an *Acremonium* cell. In another even more preferred embodiment, the filamentous fungal host cell is a *Fusarium* cell. In another even more preferred embodiment, the filamentous fungal host cell is a *Humicola* cell. In another even more preferred embodiment, the filamentous fungal host cell is a *Mucor* cell. In another even more preferred embodiment, the filamentous fungal host cell is a *Myceliophthora* cell. In another even more preferred embodiment, the filamentous fungal host cell is a *Neurospora* cell. In another even more preferred embodiment, the filamentous fungal host cell is a *Penicillium* cell. In another even more preferred embodiment, the filamentous fungal host cell is a *Thielavia* cell. In another even more preferred embodiment, the filamentous fungal host cell is a *Tolypocladium* cell. In another even more preferred embodiment, the filamentous fungal host cell is a *Trichoderma* cell. In a most preferred embodiment, the filamentous fungal host cell is an *Aspergillus awamori, Aspergillus foetidus, Aspergillus japonicus, Aspergillus aculeatus, Aspergillus niger, Aspergillus nidulans* or *Aspergillus oryzae* cell. In another preferred embodiment, the filamentous fungal host cell is a *Fusarium* cell of the section Discolor (also known as the section *Fusarium*). For example, the filamentous fungal parent cell may be a *Fusarium bactridioides, Fusarium cerealis, Fusarium crookwellense, Fusarium culmorum, Fusarium graminearum, Fusarium graminum, Fusarium heterosporum, Fusarium negundi, Fusarium reticulatum, Fusarium roseum, Fusarium sambucinum, Fusarium sarcochroum, Fusarium sulphureum*, or *Fusarium trichothecioides* cell. In another prefered embodiment, the filamentous fungal parent cell is a *Fusarium* strain of the section Elegans, *e.g., Fusarium oxysporum*. In another most preferred embodiment, the filamentous fungal host cell is a *Humicola insolens* or *Humicola lanuginosa* cell. In another most preferred embodiment, the filamentous fungal host cell is a *Mucor miehei* cell. In another most preferred embodiment, the filamentous fungal host cell is a *Myceliophthora thermophilum* cell. In another most preferred embodiment, the filamentous fungal host cell is a *Neurospora crassa* cell. In another most preferred embodiment, the filamentous fungal host cell is a *Penicillium purpurogenum* or *Penicillium funiculosum* (WO 00/68401) cell. In another most preferred embodiment, the filamentous fungal host cell is a *Thielavia terrestris* cell. In another most preferred embodiment, the *Trichoderma* cell is a *Trichoderma harzianum, Trichoderma koningii, Trichoderma longibrachiatum, Trichoderma reesei* or *Trichoderma viride* cell.

In a particular embodiment the filamentous host cell is an *A. oryzae* or *A. niger* cell.

In a preferred embodiment of the invention the host cell is a protease deficient or protease minus strain.

This may e.g. be the protease deficient strain *Aspergillus oryzae* JaL 125 having the alkaline protease gene named *"alp"* deleted. This strain is described in WO 97/35956 (Novozymes), or EP patent no. 429,490, or the TPAP free host cell, in particular a strain of *A. niger*, disclosed in WO 96/14404. Further, also host cell, especially *A. niger* or *A. oryzae,* with reduced production of the transcriptional activator (prtT) as described in WO 01/68864 is specifically contemplated according to the invention.

### Transformation of fungi

Fungal cells may be transformed by a process involving protoplast formation, transformation of the protoplasts, and regeneration of the cell wall in a manner known *per se.* Suitable procedures for transformation of *Aspergillus* host cells are described in EP 238 023 and Yelton et al., 1984, Proceedings of the National Academy of Sciences USA 81: 1470-1474. Suitable methods for transforming *Fusarium* species are described by Malardier et al., 1989, Gene 78: 147-156 and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, Journal of Bacteriology 153: 163; and Hinnen et al., 1978, Proceedings of the National Academy of Sciences USA 75: 1920.

### Methods of Production

The present invention also relates to expression of the modified nucleic acid sequence in order to produce the serum albumin. Expression comprises (a) cultivating a filamentous fungus expressing the serum albumin from the modified nucleic acid sequence; and (b) recovering the polypeptide. Preferably, the filamentous fungus is of the genus *Aspergillus,* and more preferably *Aspergillus oryzae* or *Aspergillus niger.*

In the production methods of the present invention, the cells are cultivated in a nutrient medium suitable for production of the polypeptide using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g*., in catalogues of the American Type Culture Collection). If the polypeptide is secreted into the nutrient medium, the polypeptide can be recovered directly from the medium. If the polypeptide is not secreted, it can be recovered from cell lysates.

The polypeptides may be detected using methods known in the art that are specific for the polypeptides, such as N-terminal sequencing of the polypeptide. These detection methods may include use of specific antibodies. The resulting polypeptide may be recovered by methods known in the art. For example, the polypeptide may be recovered from the nutrient medium by conventional procedures including, but not limited to, centrifugation, filtration, extraction, spray-drying, evaporation, or precipitation.

The polypeptides of the present invention may be purified by a variety of procedures known in the art including, but not limited to, chromatography (*e.g*.*,* ion exchange, affinity, hydrophobic, chromatofocusing, and size exclusion), electrophoretic procedures (*e.g*., preparative isoelectric focusing), differential solubility (*e.g*., ammonium sulfate precipitation), SDS-PAGE, or extraction (see, *e.g.,* Protein Purification, J.-C. Janson and Lars Ryden, editors, VCH Publishers, New York, 1989).

In a further aspect the present invention relates to a modified nucleic acid sequence encoding the BSA protein and capable of expression in a filamentous fungal host organism, which modified nucleic acid sequence is obtainable by:
i) providing the wild type nucleic acid sequence encoding BSA;
ii) modifying at least one codon, wherein the modification does not change the amino acid encoded by said codon and the nucleic acid sequence of said codon is different compared to the corresponding codon in the wild type gene.

In an even further aspect the invention relates to a modified nucleic acid sequence encoding the HSA protein and capable of expression in a filamentous fungal host organism, which modified nucleic acid sequence is obtainable by:
i) providing the wild type nucleic acid sequence encoding HSA;
ii) modifying at least one codon, wherein the modification does not change the amino acid encoded by said codon and the nucleic acid sequence of said codon is different compared to the corresponding codon in the wild type gene.

Particularly the wild type sequences encoding BSA or HSA are the specific sequences shown in SEQ ID NO: 1 and 3.

In the present context the term "capable of expression in a filamentous host" means that the yield of the serum albumin protein should be at least 1.5 mg/l, more particularly at least 2.5 mg/l, more particularly at least 5 mg/l, more particularly at least 10 mg/l, even more particularly at least 20 mg/l, or more particularly 0.5 g/L, or more particularly 1 g/L, or more particularly 5 g/L, or more particularly 10 g/L, or more particularly 20 g/L.

Specific examples of modified nucleic acid sequences encoding serum albumin and modified according to the invention in order to provide expression of the serum albumin protein in a filamentous fungal host, like e.g. *Aspergillus,* are shown in SEQ ID NO: 5 (BSA) and 7 (HSA). The information disclosed herein will allow the skilled person to isolate other modified nucleic acid sequences following the directions above, which sequences can also be expressed in filamentous fungi and such sequences are also comprised within the scope of the present invention.

In one embodiment the present invention relates to a modified nucleic acid sequence according to the invention as shown in SEQ ID NO: 5.

In another embodiment the present invention relates to a modified nucleic acid sequence according to the invention as shown in SEQ ID NO: 7.

Variations in the choice of codon usage and the number of codons, which have been optimized can vary and still provide as nucleic acid sequence capable of expression in a filamentous fungi. Such alternative sequences will be homologous to the specific sequences shown in SEQ ID NO: 5 and 7.

In a further embodiment the invention therefore relates to a modified nucleic acid sequence encoding the wild type BSA protein and capable of expression in a filamentous fungal host organism, wherein:
a) the modified sequence has at least 77% identity with SEQ ID NO: 5; or
b) the modified sequence hybridizes under high stringency conditions with a polynucleotide probe consisting of the complementary strand of nucleotides 1 to 1821 of SEQ ID NO: 5.

In still another embodiment the invention relates to a modified nucleic acid sequence encoding the wild type HSA protein and capable of expression in a filamentous fungal host organism, wherein:
a) the modified sequence has at least 77% identity with SEQ ID NO: 7; or
b) the modified sequence hybridizes under high stringency conditions with a polynucleotide probe consisting of the complementary strand of nucleotides 1 to 1827of SEQ ID NO: 7.

The modified nucleic acid sequence according to the invention has at 77 % identity with the sequence shown in SEQ ID NO: 5, particularly at least 79% identity, more particularly at least 82 %, more particularly at least 85 %, more particularly at least 90 %, more particularly at least 95 %, more particularly at least 98%, even more particularly at least 99 % identity.

The modified nucleic acid sequence according to the invention has at 77 % identity with the sequence shown in SEQ ID NO: 7, particularly at least 79% identity, more particularly at least 82 %, more particularly at least 85 %, more particularly at least 90 %, more particularly at least 95 %, more particularly at least 98%, even more particularly at least 99 % identity.

### Identity:

In the present context, the homology between two amino acid sequences or between two nucleic acid sequences is described by the parameter "identity".
For purposes of the present invention, alignments of sequences and calculation of homology scores may be done using a full Smith-Waterman alignment, useful for both protein and DNA alignments. The default scoring matrices BLOSUM50 and the identity matrix are used for protein and DNA alignments respectively. The penalty for the first residue in a gap is -12 for proteins and -16 for DNA, while the penalty for additional residues in a gap is -2 for proteins and - 4 for DNA. Alignment may be made with the FASTA package version v20u6 (W. R. Pearson and D. J. Lipman (1988), "Improved Tools for Biological Sequence Analysis", PNAS 85:2444-2448, and W. R. Pearson (1990) "Rapid and Sensitive Sequence Comparison with FASTP and FASTA", Methods in Enzymology, 183:63-98).

Multiple alignments of protein sequences may be made using "ClustalW" (Thompson, J.D., Higgins, D.G. and Gibson, T.J. (1994) CLUSTAL W: improving the sensitivity of progressive multiple sequence alignment through sequence weighting, positions-specific gap penalties and weight matrix choice. Nucleic Acids Research, 22:4673-4680). Multiple alignments of DNA sequences may be done using the protein alignment as a template, replacing the amino acids with the corresponding codon from the DNA sequence.

### Hybridization

For purposes of the present invention, hybridization indicates that the nucleic acid sequence hybridizes to a labeled polynucleotide probe which hybridizes to the nucleic acid sequence shown in SEQ ID NO: 5 or 7 under very low to very high stringency conditions. Molecules to which the polynucleotide probe hybridizes under these conditions may be detected using X-ray film or by any other method known in the art. Whenever the term "polynucleotide probe" is used in the present context, it is to be understood that such a probe contains at least 15 nucleotides.

In one embodiment, the polynucleotide probe is the complementary strand of SEQ ID NO: 5 or 7.

For long probes of at least 100 nucleotides in length, very low to very high stringency conditions are defined as pre-hybridization and hybridization at 42°C in 5X SSPE, 1.0% SDS, 5X Denhardt's solution, 100 µg/ml sheared and denatured salmon sperm DNA, following standard Southern blotting procedures. Preferably, the long probes of at least 100 nucleotides do not contain more than 1000 nucleotides. For long probes of at least 100 nucleotides in length, the carrier material is finally washed three times each for 15 minutes using 2 x SSC, 0.1% SDS at 42°C (very low stringency), preferably washed three times each for 15 minutes using 0.5 x SSC, 0.1 % SDS at 42°C (low stringency), more preferably washed three times each for 15 minutes using 0.2 x SSC, 0.1% SDS at 42°C (medium stringency), even more preferably washed three times each for 15 minutes using 0.2 x SSC, 0.1% SDS at 55°C (medium-high stringency), most preferably washed three times each for 15 minutes using 0.1 x SSC, 0.1% SDS at 60°C (high stringency), in particular washed three times each for 15 minutes using 0.1 x SSC, 0.1 % SDS at 68°C (very high stringency).

Although not particularly preferred, it is contemplated that shorter probes, e.g. probes which are from about 15 to 99 nucleotides in length, such as from about 15 to about 70 nucleotides in length, may also be used. For such short probes, stringency conditions are defined as prehybridization, hybridization, and washing post-hybridization at 5°C to 10°C below the calculated Tₘ using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

For short probes which are about 15 nucleotides to 99 nucleotides in length, the carrier material is washed once in 6X SCC plus 0.1 % SDS for 15 minutes and twice each for 15 minutes using 6X SSC at 5°C to 10°C below the calculated T_{m.}

### Albumin as a dried product

The present invention relates in a further embodiment to a dried and fast dissolving particle comprising serum albumin, hereinafter referred to as SA, as well as to various compositions and articles comprising the dry fast dissolving particle or said compositions of this embodiment. The present invention further relates to methods for the preparation of fast dissolving SA particle of the invention, and uses thereof.

The SA can be any form of serum albumin like e.g. human serum albumin or bovine serum albumin, and the SA species can be natural or recombinant serum albumin.

We have surprisingly found that by spray drying we obtain a product wherein the SA does not change its properties during the process step. However during simple spray drying primary particles with a very low particle size are obtained. Said primary particles can not readily disperse in liquids. The primary particles stick together and make a gel like substance when added to a liquid. To get the primary particles dispersed mechanical stirring over a prolonged period of time is required. Thus it would be desirable to obtain a SA product which readily disperses when added to a liquid and is thus fast dissolving.

In our search to find a dried powder of SA which dissolved instantly in aqueous solutions, we have surprisingly found that by enlarging the SA particles, e.g. by agglomerating primary particles of SA, we obtain a product which is readily soluble in aqueous solutions.

A "primary particle" of SA is a particle which has particle size less than 10 microns and e.g. has been prepared by spray drying one droplet of liquid.

An "agglomerate" is defined as a particle comprising at least two primary particles bonded together.

The "dispersibility" is measured in the following way: A dry HSA product corresponding to 1.0 g of pure HSA (or other serum albumin) is added to 100 ml of water (25°C) in a 600 ml beaker during gentle manual stirring with a spoon. The time for complete dissolution is measured in seconds and used as the dispersibility.

The "solubility" is measured in the following way: A dry HSA (or other serum albumin) product corresponding to 2.5 g of pure HSA product is added to 100 ml water (25°C) and gently stirred with a spoon for 20 sec. A sample of the liquid phase is taken and the dry matter content is measured. The percentage of solids in the solution is calculated as percentage of the total added solids. A solubility of 100% corresponds to all added solids being dissolved in less than 20 sec.

The "wettability" is measured in the following way: A dry HSA (or other serum albumin) product corresponding to 0.5 g of pure HSA product is added to the surface of 100 ml water (22°C) in a 600 ml beaker. The time for complete dissolution is measured in seconds and the value is the wettability.

"Bulk density" is the mass of powder per volume measured in a cylindrical container as specified in the norm: DIN 52102.

"Tapped density" is the mass of powder per volume measured in cylindrical container which is tapped a number of times as defined in the norm: ISO 787/11.

The present invention shows that a controlled agglomeration process of a dried SA product greatly improves the solubility of the product, thus achieving very fast dissolution in a solvent, which preferably is water.

### The dried SA product

The SA product of the present invention has preferably a particle size between 10 and 1,000 microns. In a particular embodiment the particle size is between 50 to 500 microns. In a more particular embodiment the particle size is between 100 and 250 microns as determined by laser diffraction measurement of the particles suspended in isopropanol, as shown in the examples below. In a particular embodiment the particle size is above 50 microns. In a more particular embodiment the particle size is above 100 microns. In a particular embodiment the particle size is below 700 microns.

In a particular embodiment of the present invention the dried product has a particle size, 50 percentile, D₅₀, which is between 10 and 1,000 microns, preferably between 100 and 1,000 microns, more preferably between 150 and 900 microns, and even more preferably between 200 and 800 microns, as determined by laser diffraction measurement of the particles suspended in isopropanol, as shown in the examples below.

It is desirable to have a narrow span of the particles as too large particles take too long time to dissolve and too small particles stick together and are also very difficult to dissolve.

In a particular embodiment, the polydispersity of the spray dried and agglomerated product is measured as the SPAN value, which is calculated according to the following formula: SPAN = (D₉₀-D₁₀)/D₅₀. In a particular embodiment of the present invention the SPAN value is less than 2.5. In a more particular embodiment the SPAN value is less than 2.0. In an even more particular embodiment the SPAN value is less than 1.5. In a most particular embodiment the SPAN value is less than 1.2.

In the above D₁₀ is the average particle size where 10 % of the mass of particles are smaller than or equal to this size. D₅₀ is the average particle size where 50 % of the mass of particles are smaller than or equal to this size. D₉₀ is the average particle size where 90 % of the mass of particles are smaller than or equal to this size.

The amount of SA present in the dried SA agglomerate of the present invention is in a particular embodiment more than 50%. In a more particular embodiment of the present invention the SA present in the dried SA agglomerate is more than 75%. In a more particular embodiment of the present invention the SA present in the dried SA agglomerate is more than 90%. In a most particular embodiment of the present invention the SA present in the dried SA agglomerate is more than 95% even more than 99. In a particular embodiment the SA present in the dried SA agglomerate is 100%.

In a particular embodiment the particles dissolve within 5 minutes. In a more particular embodiment the particles dissolve within 3 minutes. In an even more particular embodiment particles dissolve within 1½ minutes. In a most particular embodiment the particles dissolve within ½ minutes.

The dried SA agglomerate of the present invention may also comprise other ingredients. Other ingredients may be but are not limited to fillers, agglomeration aids, one or more active ingredients, such as pharmacologically active substances, and water-soluble excipients, buffer, salts, surfactants, polymers e.g. polyethylene glycol, carbohydrates e.g. starch, mannitol, sorbitol, inorganic salt such as sodium chloride, phosphate and a mixture thereof. Nutrients for cells, such as salts and sugars for cell culture media, said other ingredient may be added to the liquid composition comprising the SA.

Further ingredients, which may be added to the liquid composition is heat stabilizers for SA e.g. octanoate or N-acetyl-tryptophan.

For all uses, the albumin may be spray-dried with a combination of one ore more compounds, including but not limited to pharmaceutical actives, e.g. to antigens, antibodies, antibody fragments, hormones, growth factors, interleukins Betaferon (interferon- -1b), human erythropoietin (EPO), glucocerebrosidase, insulin, in particular recombinant insulin produced in *E. coli and S. cerevisiae*, Urate oxidase, Glucagon, Granulocyte-macrophage colony stimulating factor, Hirudin, lepirudin, Platelet-derived growth factor, Antiangiogenic factor, Antibodies (mAb and/or PAb), antibody fragments (Fab), Anti microbial peptides, anti viral peptides, anti-cancer peptides, IGF (insulin like growth factor e.g. IGF-1, IGf-2), EGF (epidermal growth factor), TGF (TGF-alfa) Transforming growth factor, Heparin, Interleukins, Cytokines, Hormones (including but not limited to sex hormones, such as Follicle Stimulating hormone and Human chorionic gonadotrophin, human growth hormone, peptide hormones, cortison hormones), Antigens (e.g. HIV, HBV, HCV, HPV, Dengue Virus, Malaria, Hepatitis B surface antigen, Measles, Rubella, Mumps), Botulinum toxins, Elastin, Gellatine, Collagen, Protein A, Lactic acid, Hyaloronic acid, human intrinsic factor.

### The liquid composition

The liquid composition to be formulated into a dry, fast dissolving product comprises SA. The liquid composition may further comprise other ingredients.

The dry solid content of the liquid composition may vary between 1 and 30 wt%. In a particular embodiment of the present invention the dry solid content of the liquid composition varies between 3 and 15 wt %. If the dry solid content is too high the liquid composition becomes too viscous and it will be difficult to handle.

The liquid composition may comprise other ingredients. In a particular embodiment of the present invention the amount of other ingredients does not exceed 50 wt% of the dry solids. In a more particular embodiment of the present invention the amount of other ingredients does not exceed 25 wt%.

In a particular embodiment the liquid composition is aqueous.

### Method of producing dried SA particles

Normally, when spray drying particles the particles obtained are primary particles and they are very small e.g. below 10 microns. Said primary particles are very difficult to disperse and dissolve in aqueous liquid. However we have found that larger particles disperse and dissolve readily in aqueous water. We have found that it is possible to prepare enlarged particles e.g. by agglomeration of primary particles or by running the spray drying process under specific conditions whereby the droplets prepared by atomization are very big and thereby the dried particles obtained also are bigger compared to particles obtained by spray drying run under normal conditions. By using the latter process it is not necessary to include a fluid bed.

The present invention provides a method for producing a dry and fast dissolving SA product and/or preferred embodiments thereof.

In one embodiment of the present invention the method comprises the steps of:
a) drying a liquid composition comprising serum albumin; and
b) agglomerating the product of a).

In another embodiment of the present invention the method comprises the steps of
a) preparation of a liquid composition comprising serum albumin;
b) atomization of the liquid composition of step a)
c) drying of the liquid composition to obtain particles with a particle size above 50 microns.

In a particular embodiment of the present invention the atomization in step b) is performed by use of a two fluid nozzle atomizer, a pressure nozzle or a rotary atomizer.

To obtain particles with a particle size above 50 microns by use of a two fluid nozzle atomizer or a pressure nozzle the atomization pressure has to be low. In a particular embodiment of the present invention the atomization pressure is below 5 bar In a more particular embodiment of the present invention the atomization pressure is below 3 bar.

To obtain particles with a particle size above 50 microns by use of a rotary atomizer the rotation speed has to be low. In a particular embodiment the rotation speed has to be below 100 m/s. In a more particular embodiment the rotation speed has to be below 50 m/s.

Drying of the SA containing liquid composition may be achieved by any drying method available to a skilled person such as spray drying, freeze drying, vacuum drying, fluid bed drying and microwave drying. It is preferred that a fast drying method is used. If a slow drying method is used the risk of starting protein polymerization is high.

In a particular embodiment spray drying is used in the drying step as spray drying is a fast process and it is possible to add gas into the liquid formulation to vary the porosity of the obtained particles.

The drying and enlargement of the particles may take place in, but are not limited to, process equipment like spray dryers, fluid beds and or equipment including both processes wherein both are combined such as fluid bed spray dryers (fluidized spray dryer).

In a particular embodiment of the present invention a fluidization of the particles is included in the process.

In a particular embodiment the present process combines the principles of drying processes and agglomeration processes. In a particular embodiment a spray dryer and a fluid bed is used.

In a particular embodiment of the present invention these two principles are combined into one piece of equipment e.g. a fluidized spray dryer. To save process time it is convenient that the drying and agglomerating is done in one step. In a particular embodiment of the present invention the drying and agglomeration is done in a one process step by using a fluidized spray dryer. Typically the inlet temperature is between 50-200°C. The air outlet temperature is 20-90°C.

In a particular embodiment of the present invention the particles e.g. primary particles, to be re-cycled are blown into the atomization zone in the spray dryer wherein the agglomeration occurs.

In another particular embodiment the fluid bed is equipped with nozzles which are spraying the liquid composition onto the primary fluidized particles formed in the fluid bed whereby agglomerates are formed.

In a particular embodiment of the present invention the invention discloses a process for the production of agglomerated SA wherein a liquid composition is prepared comprising SA, optionally additives are added to the composition. One or more of said liquid compositions are sprayed into a fluidized bed. In a particular embodiment the liquid compositions are sprayed into a fluidized bed from below by means of spray nozzles. Fine material that escapes from the fluid bed with the off gas is separated and returned to the fluidised bed as nuclei for the agglomerates. Agglomerates of a pre-determined size are formed by adjusting the sifting gas stream and the finished agglomerates are discharged.

It may be convenient first to produce the dry fine powder by e.g. spray drying. Said fine powder is fluidized in a fluid bed and a liquid binder e.g. water is sprayed into the equipment to build up the desired agglomerates.

After drying the obtained fast dissolving SA particles/agglomerate is removed from the process.

In a particular embodiment of the present invention it is necessary to include a re-cycling system. Said recycling system may recycle primary particles or particles which in general are too small or particles which have been grinded as they are too big. The system works preferably by classifying the dried particles in two or more size classes wherein the particles which are too small are re-cycled and the particles of the desired size are removed from the process. The recycling system may work as an internal system and/or an external system.

In an external system the material to be re-cycled escaping from the fluidised bed may be continuously separated off from the off air with the aid of a cyclone separator or dust filter and returned to the fluidised bed. In an internal system the material to be re-cycled is effected with aid of a dust filter arranged above the fluidised bed.

The process may include the grinding of oversized particles and return of the grinded particles to the fluidized bed by an external system.

The recycling system may work as a wet or dry recycling system. In a wet re-cycling system the re-cycled particles are dissolved or suspended into the in-going liquid composition. In a dry re-cycling system the particles are returned back into the atomization zone.

In a particular embodiment the technique used is a spray dryer equipped with a Walzel type of atomizer and a re-cycle system.

It has been found that by introducing a gas into the particles during spray drying it is possible to vary the porosity of the obtained particles. Porous particles are normally faster dissolving than non-porous particles. It is thus possible to vary the solubility of the spray dried particles.

The process may include a process step where a gas is introduced into the liquid composition.

In a particular embodiment the gas to be introduced is carbon dioxide, nitrogen, or atmospheric air.

### Serum albumin applications

Albumin, whether obtained recombinantly or from plasma sources, can be used for a number of applications. It is known to exhibit a variety of functions both *in vitro* and *in vivo.* (Kragh-Hansen U, Chuang VT, Otagiri M (2002). Practical aspects of ligand-binding and enzymatic properties of human serum albumin. Biological & Pharmaceutical Bulletin. 25(6): 695-704; Curry S (2002). Beyond expansion: structural studies on the transport roles of human serum albumin. Vox Sanguinis. 83:Suppl-9; Nicholson JP, Wolmarans MR, Park GR (2000). Brit J Anaesth 85(4): 599-610; Peters, Theodore. All about albumin: biochemistry, genetics, and medical. Academic Press 1996, ISBN 0-12-552110-3)

In some applications, particularly in applications where there is a desire to use non-animal origin components, it is advantageous to combine recombinantly produced albumin with some or all of the following compounds when they are produced recombinantly or naturally by microbial systems: insulin, transferrin, IGF1, EGF or other proteins, growth factors and metabolites.

The spray-dried SA particles/agglomerate or/and the recombinantly produced SA according to the invention will be useful either as a final product or for production of albumin containing products used for:
- Cell culture media
- component in diagnostic kits
- stabilizer of protein solutions
- applications within the pharmaceutical area such as blood expanders and excipients
- digestive support
- removal of toxins
- imaging - radiologic or ultrasonic imaging
- drug delivery
- coating of surfaces e.g. medical devices
- invitro fertilization - both as storage medium of egg alone, sperma alone, but also for culturing of egg + sperma.

The serum albumin according to the invention can replace albumin derived from any animal species, most particular from human or bovine sources, or recombinant animal albumins, at an equivalent or better function for all uses of albumin. The reasons for this includes: 1) The invention, as it is herein described, naturally creates beneficial species of small molecules bound to the albumin molecules, including, but not limited to, molecules such as fatty acids, vitamins, amino acids, phospholipids and cations; and 2) It does not contain the high amounts of caprylic acid and N-acetyl DL tryptophan that many manufacturers of native and recombinant albumin use as stabilizers. It is well known that fatty acid and cation binding to albumin produce conformational changes which further affect both cooperative and competitive interactions of fatty acids and drugs. Excessive amounts of caprylic acid and/or N-acetyl DL tryptophan often have unwanted or adverse effects in many albumin applications. The use of recombinant human albumin in critically ill patients has thus been shown to increase mortality. (Olsen H, Andersen A, Nordbø A, Kongsgaard UE, Børmer OP, 2004. Pharmaceutical grade albumin: impaired drug binding capacity in vitro. BMC Clinical Pharmacology, 4:4 doi:10:1186/1472-6904-4-4; Keenan J, Dooley M, Pearson D, Clynes M. Recombinant human albumin in cell culture: Evaluation of growth promoting potential for NRK and Scc-9 cells in vitro. Cytotechnology 1997, 24:243-52; Zunszain, PA, Monie T, Konarev PV, Svergun DV, Curry S (2003). Structural analysis of conformational changes in human serum albumin associated with ligand binding and pH. www-hasylab.desy.de?science/annual_report/2003_report/part2/contrib./73/9952.pdf).

### Applications for SA

The applications for SA according to the present invention include but are not limited to:
**i)** the culture of mammalian cells for research, diagnostic or therapeutic purposes; the culture of genetically engineered or non-genetically engineered mammalian cells, including but not limited to CHO, Sp2/0, NSO, BHK, HEK 293, Namalwa and PERC.6, A431, for the production of biopharmaceuticals, diagnostic reagents or native or recombinant proteins, or adenoviruses to be used for medical or cosmetic purposes, and culture media for the same; the culture of normal primary human cells, for example those offered commercially from Clonetics, Cascade or Cell Applications, and culture media for the same; the culture of stem cells, for example cells available from Stem Cell Technologies or patient derived samples for bone marrow transplants or myocardial infarct repair, and culture media for the same; the culture of mammalian fibroblasts with and without kerotinocytes, for example Dermagraft © from Smith+Nephew, and the culture media for the same; the culture and expansion of mammalian tissue for implant and lesion repair, for example autologous chondrocyte implantation or myocyte implantation, and culture media for the same; (Yamane I. 1978. Development and application of a serum-free culture medium for primary culture. In H. Katsuta (ed), Nutritional Requirements of Cultured Cells. Baltimore, University Park Press, pp 1-21; US patent 5,021,349; Iscove NN, Melchers F (1978). Complete replacement of serum by albumin, transferrin and soybean lipid in cultures of lipopolysaccharide-reactive B lymphocytes. J Exp Med 147: 928-33; US patent 5,198,349; US patent 5,250,421; Berntorp E (1997). Thrombosis Haemostasis 78: 256-60; McGrew JT, Richards CL, Smidt P, Dell B and Price V. 1998. Lipid requirements of a recombinant Chineese Hamster Ovary Cell Line (CHO), ibidem, pp 205-207; Yamane I. 1978. Role of bovine albumin in a serum-free culture medium and its application. Natl Cancer Inst Monogr 48:131-133; Sato JD, Kawamoto T, McClure DB and Sato GH. 1984. Cholesterol requirement of NS-1 mouse myeloma cells for growth in serum-free medium. Mol Biol Med 2(2):121-134; Kovar J. Hybridoma cultivation in defined serum-free media: growth-supporting substances. IV. Lipids and serum albumin. Folia Biol (Praha). 1987, 33(6):377-84; Jaeger, V, Lehmann J, Friedl P. Serum-free growth medium for the cultivation of a wide spectrum of mammalian cells in stirred bioreactors. Cytotechnology 1988, 1:319-29; Glassy CM, Tharakan JP, Chau, PC. Serum-free media in hybridoma culture and monoclonal antibody production. Biotech Bioeng 1988, 32:1015-28).
**ii)** use as a cryoprotectant for mammalian cells; ( Somlo G, et al (1997). Effect of CD34+ selection and various schedules of stem cell reinfusion and granulocyte colony stimulating factor priming on hematopoetic recovery after high-dose chemotherapy for breast cancer. Blood 89: 1521-8; US patent 6,548,297; WO01/37655; JRH Biosciences Catalog, 2004. Section on general cell culture techniques.
**iii)** use in or for process solutions used in mammalian assisted reproduction techniques; (Armstrong JS, Rajasekaran M, Hellstom WJG, Sikka SC (1998). Antioxidant potential of human serum albumin: role in recovery of high quality spermatozoa for assisted reproductive technology. J Androl 19:412-9; VandeVoort CA (2004). High quality sperm for non-human primate ART: Production and assessment. Reproduct Biol Endocrinol, 2: 33-8; Lane M, Maybach JM, Hooper K, Hasler JF, Gardner DK (2002). Cryo-survival and development of bovine blastocysts are enhanced by culture with recombinant albumin and hyaluronan Molecular Reproduction & Development 64: 70-8; Gardner, DK (2004). US patent 6762053. Mammalian gamete and embryo culture media and culture media supplements.
**iv)** use in solutions for the preservation of donor organs; (US patent application 20040029096).
**v)** use in ocular applications; (Shimmura S, Ueno R, Matsumoto Y, Goto E, Higuchi A, Shimazaki J, Tsubota K (2003). Albumin as a tear supplement in the treatment of severe dry eye. Brit J Ophthalmology 87:1279-83; US patent 6,043,213).
**vi)** use in therapeutic applications as a plasma expander or for osmotic control, similar to the products Buminate (Baxter Intl), Plasbumin (Bayer Corp.) and Hextend (BioTime); (Woodruff LM, Gibson ST (1942). The clinical evaluation of human albumin. US Navy Med Bull 40:791-6; Heyl JT, Gibson JG II, Janeway CA (1943). Studies on the plasma proteins. V. The effect of concentrated solutions of human and bovine serum albumin on blood volume after acute blood loss in man. J Clin Invest 22: 763-73; Alderson P, Bunn F, Lefebvre C, Li Wn Po A, Li L, Roberts I, Schierhout G (2004). Human albumin solutions for resuscitation and volume expansion in critically ill patients. The Cochrane Database of Systematic Reviews, Issue 4. Art No. CD001208.pub2.DOI: 10.1002/14651858.CD001208.pub2)
**vii)** use as an excipient in the manufacture or formulation of pharmaceuticals or as a carrier, protecting agent, stabilizer or other use involving non-covalent association of another molecule, such as a drug, peptide or protein with the albumin (for example albumin-bound paclitxel suspension), for diagnostic or therapeutic use, including hormones (for example IGF-1 or insulin) and cytokines; (Tarelli E, et al (1998). Recombinant human albumin as a stabilizer for biological materials and for the preparation of international reference reagents. Biologicals 26: 331-46; Paul W, Sharma CP (2005). Bioceramics, Towards Nano-enabled Drug Delivery: A mini Review. Trends Biomater. Artif Organs, 19: 7-11; Roddie, PH, Ludlam CA (1997). Blood Reviews 11:169-77).
**viii)** use in cosmetics or medical cosmetic procedures; (Sidle DM, Loos BM, Ramirez AL, Kabaker SS, Maas CS (2005). Use of BioGlue Surgical Adhesive for brow fixation in endoscope browplasty. Arch Facial Plast Surg 7: 393-7).
**ix)** use for inclusion in, on, or in the manufacturing of medical devices, including dental or dental implant applications, bone repair materials and biocompatible substances; (Kinnari TJ, Rihkanen H, Laine T, Salonen, E-M, Jero, J (2004). Albumin-coated tympanostomy tubes: Prospective, double-blind clinical study. Laryngoscope 114: 2038-43; US patent application 20030004105.
**x)** use as a reagent in diagnostic procedures, kits or methods, for the purposes including but not limited to blocking non-specific adsorption of substances to surfaces, for local pH and osmolarity control in solution, to increase temperature stability of diagnostic or assay reagents, as a non-specific enzyme or small molecule stabilizer, as a stabilizer in the freezing or freezedrying of small molecule, peptide and protein reagents.
**xi)** in the manufacture of human or veterinary vaccines, for example in Merck's MMR-II and MUMPSVAX vaccines, rabies vaccines, hepatitis A vaccine, the immunostabilization of virus in polymerized albumin; (US patent 6,884,422; The BSE Inquirey: The Report (2000). Volume 16 chapter 4. www. bseinquirey.gov.uk).
**xii)** use as a standard or reference material; (Bradford M (1976). A rapid and sensitive method for the quantitation of microgram quantities of protein utilizing the principle of dye-binding. Anal Biochem 72: 248-54).
**xiii)** use in or for adhesives or sealants, similar to CryoLife BioGlue © or albumin fixatives; (Fuerst W, Banerjee A (2005). Release of glutaraldehyde from an albumin-glutaraldehyde tissue adhesive causes significant in vitro and in vivo toxicity. Ann Thorac Surg 79:1522-8; Passage J, Tam R, Windsor M, O'Brien M (2005). BioGlue: A review of the use of this new surgical adhesive in thoractic surgery. ANZ J Surg 75:315-8; Hoffman GT, et al (2004). Composites containing albumin protein or cyanoacrylate adhesives and biodegradable scaffolds: I. Acute wound closure study in a rat model. Proc SPIE, 5312:117-23); Medical Adhesives & Sealants (2003). Study #1681. The Freedonia Group).
**xiv)** use in the removal of toxins (Cole and Lirenman, 1978, J. Pediatr. 92:955-957)
**xv)** all other human or non-human applications and uses where an extracted or a recombinant mammalian albumin could be employed;

### Loading of serum albumin

However, in some applications, particularly applications where albumin is used as a cell culture ingredient, it is advantageous to load the albumin molecule with one or more ligands, including but not limited to fatty acids, vitamins, hormones and ions - e.g. cupper, zink etc.

In some applications, it is advantageous to process albumin. Examples of such processing are:
- Chromatographic purification, such as cat- and anion-exchange chromatography, affinity chromatography, reverse phase chromatography and mixed mode chromatography. The chromatography mode may, e.g., be packed bed or expanded bed adsorption/affinity chromatography.
- Treatment with activated carbon and dextran coated charcoal or solvent extraction
- Heating or pasteurisation in the presence of a stabilizer, e.g. caprylic acid and/or N-acetyltryptophan
- Ultrafiltration
- Protection of the protein from proteolytic degradation by addition of protease inhibitors.

### Using these and other techniques, it may be possible to:

Reduce the colour of albumin, strip off non-covalently bound ligands, remove unwanted impurities, *e.g*. nucleic acids, enzyme activities - such as protease activities - carbohydrates, endotoxins and host cell protein as well as albumin derived impurities. The invention can be modified further by depleting the molecules that are not covalently bound to it and then reconstituting the albumin bound molecules specifically for the intended application to ensure a more optimum function. For cell cultures applications, albumin which is depleted of small molecules that remain bound from the manufacturing process has been shown to perform better for a given application when it is reconstituted with physiologically relevant mixtures of free fatty acids, phospholipids, cholesterols, hormones, metal ions, vitamins or drugs, more specifically one or more of linoleic acid, oleic acid, cholesterols, folic acid, cobalamin, pyridoxine, thyroxine, calcium (II)copper (II) and zinc (II).

(Rumsey SC, Galeano NF, Lipschitz B, Deckelbaum RJ. (1995) J Biol Chem 270:10008-16; Bhattacharya AA, Curry S, Franks NP. Binding of the general anesthetics propofol and halothane to human serum albumin: high resolution crystal structures. J Biol Chem 2000, 275:38731-8; Bhattacharya AA, Gruene T, Curry S. Crystallographic analysis reveals common modes of binding medium and long-chain fatty acids to human serum albumin. J Mol Bio 2000, 303:721-32. Synopsis: Similar to the method in C.2.2; Petitpas I, Gruene T, Bhattacharya AA, Curry, S. Crystal structures of human serum albumin complexed with monounsaturated and polyunsaturated fatty acids. J Mol Bio 2001, 314:955-60; Li Z, Zhuang J, Corson DW. Delivery of 9-Cis retinal to photoreceptors from bovine serum albumin. Photochem Photobiol. 1999, 69(4):500-4. Albumin is also known to carry T4, folate and vitamin B12. Albumin-related gene products are known to carry vitamin D and vitamin K; Viscardi RM, Ullsperger S, McKenna MC. Carbon stripping extracts serum free fatty acids: implications for media supplementation of cultured type II pneumocytes. Lab Invest. 1991, 65(2):250; Caro JF, Hodges J, Sinha MK. Increased insulin responsiveness in isolated rat hepatocytes incubated with free fatty acid-poor albumin. Horm Metab Res. 1991, 23(8):362-4).

Moreover, in some applications, it is advantageous to enrich for certain covalent modifications of albumin - either by purification, chemically or biological strategies. Such modifications include reacting mercaptalbumin with cysteine or glutathione and using a host that predominantly produces a desired species rSA. Albumin blocked by cysteine or glutathione have improved heat stability, which is advantageous in certain applications or processing steps, e.g. in connection with heat denaturation of unwanted proteins - particularly unwanted enzymes.

Surprisingly, rSA produced by *Aspergillus oryzae* by recombinant expression, was found to support growth of mammalian cells in *in vitro* cultures - without prior loading - to an extent that exceeds that of unloaded as well as naturally loaded nSA. Even more surprisingly, HSA incubated with extracts from *Aspergillus oryzae* resulted in albumin that supports proliferation to an even greater extent. It is generally accepted that loading albumin with fatty acids confers a growth and often also productivity enhancing effect to mammalian cell cultures. rHSA from *Aspergillus oryzae* was surprisingly found to be loaded with linoleic acid, which is an essential fatty acid that in many applications will provide a positive contribution to growth and productivity of cell cultures. Some or all of these performance enhancing properties of rHSA from filamentous fungi can be further amplified by incubating albumin preparations, from any source, with extracts from filamentous fungi.

Serum albumin contacted with extracts derived from cultures of filamentous fungi will exhibit a growth- and probably also productivity-enhancing effect when added to cell culture medium.

Serum albumin loaded as described above is modified in a way that is particularly useful for application in cell culture medium, more particularly in serum free cell culture medium for primary cultures.

"Loading" in the context of the present invention means any modification to the mature serum albumin polypeptide, which includes addition of fatty acids but could also include addition of other factors, which modification takes place either when the serum albumin is expressed in a filamentous fungi, particularly an *Aspergillus* sp, or when any serum albumin, native, recombinant or fatty acid free, is contacted with a lysate derived from a culture of a filamentous fungi.

In one aspect the invention therefore relates to a loaded serum albumin obtainable by:
i) recombinant expression of a nucleic acid sequence encoding the serum albumin in a filamentous fungal host cell; and/or
ii) loading the serum albumin by contacting said serum albumin with a cell extract derived from filamentous fungal cells.

The loading can either be achieved by the recombinant expression of serum albumin in a suitable host cell or by contacting serum albumin from any source with a cell extract derived from a filamentous fungus.

The cell extract can in one embodiment be obtained by lysing the filamentous fungal host cells. It is obvious to the skilled person that it is most efficient to use a cell lysate whereby the cells are actively disrupted, however, due to secretion and spontaneous lysis of cells in culture it is very likely that the culture medium as such could also be applied for loading serum albumin.

In a particular embodiment loading is achieved by contacting the serum albumin with culture broth used for culturing a filamentous fungus.

The term "cell extract" in the present context thus comprises culture broth from a cell culture of a filamentous fungi as well as a lysate obtained from a cell culture of a filamentous fungi or even a combination of both.

The filamentous fungal host cell is in one embodiment selected from the group consisting of *Acremonium, Aspergillus, Fusarium, Humicola, Mucor, Myceliophthora, Neurospora, Penicillium, Thielavia, Tolypocladium*, or *Trichoderma*.

Particularly the *Aspergillus* cell is *Aspergillus awamori, Aspergillus foetidus, Aspergillus japonicus, Aspergillus niger, Aspergillus nidulans*, or *Aspergillus oryzae.*

In one particular embodiment the loaded serum albumin is HSA or BSA.

In another particular embodiment the HSA or BSA is recombinantly produced in the filamentous fungi from the nucleic acid sequences selected from the group consisting of SEQ ID NO 5 and SEQ ID NO 7.

Loaded serum albumin according to the invention has been shown to be very useful when used in cell culture medium, thereby improving parameters such as cell viability, cell growth, and/or productivity.

The amount of SA added to cell culture medium is usually in the range from 0.1-100.000 µg/ml, particularly from 1-10.000 µg/ml, more particularly from 100-10.000 µg/ml medium.

In one embodiment the invention relates to a use of loaded serum albumin according to the invention in a cell culture medium. Particularly in serum free cell culture medium.

### MATERIALS AND METHODS

### Materials

### Strains

MBin115 is described in WO 2004/090155, example 9.

### Example 1. Cloning of the gene encoding Human Serum albumin (HSA)

HSA has many applications such as media component or for drug delivery. It is of interest to see if HSA can be expressed at economically interesting levels by the currently used hosts, especially the filamentous fungi.

Human adult male liver first strand cDNA was ordered and received from Stratagene (cat. no. 780621). The following two DNA oligo's where ordered and received:
190203J1
   SEQ ID NO: 9: ATGGACGGATCCACAATGAAGTGGGTAACCTTTATTTCC
190203J2
   SEQ ID NO: 10: ATGGACCCGCGGCTCGAGTTATAAGCCTAAGGCAGCTTGACTTGC

The following PCR was run using the two DNA oligoes and the cDNA as template along with the Pwo-polymerase (Roche); 94°C 5 min 25* (94°C 30 sec, 55°C 30 sec, 72°C 3 min) 72°C 7 min.

The resulting PCR fragment was cloned into pCR4 blunt using the TOPO kit as recommended by manufacture (Invitrogen, cat no. 601059) resulting in plasmid pEN13046.

The gene was sequenced (see sequence at the end)

The cloned HSA has no N-glycosylation site.

The plasmid pEN13046 was cut with BamHI and Sacll and the gene was isolated from agarose gel. The plasmid pEN12516 (WO 2004/069872) was cut BamHl and Sacll and isolated from agarose gel. The vector and the gene was ligated and transformed into DH10b. The resulting plasmid was named pEN13054.

### Example 2. Transformation of HSA cDNA expression plasmid into Aspergillus oryzae

The plasmid pENI3054 was transformed in to the *Aspergillus oryzae* strain JAI355 (WO 2004/069872).This was done as mentioned in WO 2004/069872.

10 transformants were grown in 200 µl YPM in a 96 well microtiter dish for 3 days at 34°C.

20 µl of supernatant was run on SDS-PAGE to see if the HSA was expressed by *A.oryzae.* No bands were detectable.

### Example 3. Transformation of HSA cDNA expression plasmid into Aspergillus niger

The plasmid pEN13054 was transformed in to the *Aspergillus niger* strain MBin115 (which was prepared as described in WO2004/090155, example 9).

10 transformant were grown in 200 µl YPM in a 96 well microtiter dish for 3 days at 34°C.

20 µl of supernatant was run on SDS-PAGE to see if the HSA was expressed by *A.niger.*

No bands were detectable.

### Example 4. Cloning of BSA encoding gene

BSA has many applications such as media component or for drug delivery. It is of interest to see if BSA can be expressed at economically interesting levels by the currently used hosts, especially the filamentous fungi.

Bovine liver cDNA library was ordered and received from Stratagene (cat. no. 937712). The following two DNA oligo es where ordered and received:
150503j6
   SEQ ID NO: 11: GACTCGGGATCCACAATGAAGTGGGTGACTTTTATTTCTC
150503j7
   SEQ ID NO: 12: GACTCGCCGCGGCTCGAGTTAGGCTAAGGCTGTTTGAGTTGA

The following PCR was run using the two DNA oligoes and the cDNA as template along with the Pwo-polymerase (Roche); 94°C 5 min 25* (94°C 30 sec, 55°C 30 sec, 72°C 3 min) 72°C 7 min.

The plasmid resulting PCR fragment was cut with BamHl and Sacll and the gene was isolated from agarose gel. The plasmid pENI2516 (WO 2004/069872) was cut BamHl and Sacll and isolated from agarose gel. The vector and the gene was ligated and transformed into DH10b. The resulting plasmid was named pENI3113.

The gene was sequenced (see sequence at the end).

The cloned BSA has no N-glycosylation site.

### Example 5. Transformation of BSA cDNA expression plasmid into Aspergillus oryzae

The plasmid pENI3113 was transformed in to the *Aspergillus oryzae* strain JAL355 (WO 2004/069872).This was done as mentioned in WO 2004/069872.

10 transformant were grown in 200 µl YPM in 96 well microtiter dish for 3 days at 34°C.

20 µl of supernatant was run on SDS-PAGE to see if the BSA was expressed by *A. oryzae.*

No bands were detectable.

### Example 6. Transformation of BSA cDNA expression plasmid into Aspergillus niger

The plasmid pEN13113 was transformed in to the *Aspergillus niger* strain Mbin115. This was done as mentioned in WO 2004/069872.

10 transformant were grown in 200 µl YPM in 96 well microtiter dish for 3 days at 34°C.

20 µl of supernatant was run on SDS-PAGE to see if the BSA was expressed by *A. niger.*

No bands were detectable.

### Example 7. Construction of a HSA Aspergillus expression plasmid based on the synthetic gene

The Aspergillus expression plasmid pJaL721 (WO 2003/008575) consist of an expression cassette based on a mutated version the Aspergillus niger neutral amylase II promoter and the *Aspergillus niger* amyloglycosidase terminater (Tamg). Also present on the plasmid is the *Aspergillus* selective marker *amdS* from *Aspergillus nidulans* enabling growth on acetamide as sole nitrogen source and the URA3 marker from *Saccharomyces cerevisiae* enabling growth of the *pyrF* defective *Escherichia coli* strain DB6507 (ATCC 35673).

Transformation into *E. coli DB6507* using the S. cerevisiae URA 3 gene as selective marker was done in the following way:
E. coli DB6507 was made competent by the method of Mandel and Higa (Mandel, M. and A. Higa (1970) J. Mol. Biol. 45, 154). Transformants were selected on solid M9 medium (Sambrook et. al (1989) Molecular cloning, a laboratory manual, 2. edition, Cold Spring Harbor Laboratory Press) supplemented with 1 g/I casaminoacids, 500 µg/l thiamine and 10 mg/l kanamycin.

The synthetic HSA gene was cloned into pJaL721 in the following way:
The synthetic gene was purchased from DNA2.0, Menlo Park, CA, USA (www. Dnatwopointo.com) and received on the plasmid pDrive-G0035R. This plasmid was digested with the restriction enzymes BamH I and Sal I and the 1842 bp fragment harboring the HSA gene was purified from an agarose gel.

This fragment was ligated to pJaL721 digested with BamH I and Xho I. The ligation mixture was transformed into *E. coli* DB6507. A plasmid from one of the colonies formed was confirmed to have the expected insert by restriction analysis and DNA sequencing. This plasmid was termed pCaHj620. A restriction map of pCaHj620 is shown in figure 1.

### Example 8. Transformation of the synthetic HSA expression plasmid into Aspergillus oryzae

pCaHj620 was transformed into Aspergillus oryzae BECh2 (WO 00/39322, example 1), a number of Transformants were spore reisolated twice. Spores from second reisolation of each transformant were used to inoculate 10 ml YPM (1% yeast extract, 2% peptone, 2% maltose) in 25 ml NUNC containers. The YPM cultures were grown for 4 days at 34°C under orbital shaking, and the supernatants were applied to SDS polyacrylamide gels (Bio-Rad, Criterion XT precast gels, Bio-Rad Laboratories, Hercules, CA, USA) and stained with coomassie blue stain using standard methods. A band of approx. 67 kDal was observed in different amounts from the transformants. The highest yielding transformants were selected for laboratory tank fermentation.

### Example 9. Transformation of the synthetic HSA expression plasmid into Aspergillus niger

pCaHj620 was transformed into Aspergillus niger MBin118 (WO 2004/090155, Aspergillus niger clean host), a number of Transformants were spore reisolated twice. Spores from second reisolation of each transformant were used to inoculate 10 ml YPM (1% yeast extract, 2% peptone, 2% maltose) in 25 ml NUNC containers. The YPM cultures were grown for 4 days at 34°C under orbital shaking, and the supernatants were applied to SDS polyacrylamide gels (Bio-Rad, Criterion XT precast gels, Bio-Rad Laboratories, Hercules, CA, USA) and stained with coomassie blue stain using standard methods. A band of approx. 67 kDal was observed in different amounts from the transformants.

### Example 10. Construction of a BSA Aspergillus expression plasmid based on the synthetic gene

The synthetic BSA gene was cloned into pJaL721 in the following way:
The synthetic gene was purchased from GenScript Corrporation (Scotch Plains, NJ) (www.genscript.com) and received as a plasmid. This plasmid was digested with the restriction enzymes BamH I and Xho I and the 1837 bp fragment harboring the HSA gene was purified from an agarose gel.

This fragment was Iligated to pJaL721 digested with BamH I and Xho I. The ligation mixture was transformed into *E. coli* DB6507. A plasmid from one of the colonies formed was confirmed to have the expected insert by restriction analysis and DNA sequencing. This plasmid was termed pCaHj623. A restriction map of pCaHj623 is shown in figure 2.

### Example 11. Transformation of the synthetic BSA expression plasmid into Aspergillus oryzae

pCaHj623 was transformed into Aspergillus oryzae BECh2, a number of Transformants were spore reisolated twice. Spores from second reisolation of each transformant were used to inoculate 10 ml YPM (1% yeast extract, 2% peptone, 2% maltose) in 25 ml NUNC containers. The YPM cultures were grown for 4 days at 34°C under orbital shaking, and the supernatants were applied to SDS polyacrylamide gels (Bio-Rad, Criterion XT precast gels, Bio-Rad Laboratories, Hercules, CA, USA) and stained with coomassie blue stain using standard methods. A band of approx. 67 kDal was observed in different amounts from the transformants. The highest yielding transformants were selected for laboratory tank fermentation.

### Example 12. Transformation of the synthetic BSA expression plasmid into Aspergillus niger

pCaHj623 was transformed into Aspergillus niger MBin118, a number of Transformants were spore reisolated twice. Spores from second reisolation of each transformant were used to inoculate 10 ml YPM (1% yeast extract, 2% peptone, 2% maltose) in 25 ml NUNC containers. The YPM cultures were grown for 4 days at 34°C under orbital shaking, and the supernatants were applied to SDS polyacrylamide gels (Bio-Rad, Criterion XT precast gels, Bio-Rad Laboratories, Hercules, CA, USA) and stained with coomassie blue stain using standard methods. A band of approx. 67 kDal was observed in different amounts from the transformants.

### Example 13. Lading of commercial fatty acid free HSA with lysed Aspergillus oryzae cell extract.

- Fermentation broth (including cells) from *Aspergillus oryzae* are treated with ultrasound on ice (4 × 30 seconds).
- Centrifugation @ 4000 × g, 5°C, 30 minutes. Supernatant is filtered using 0.22 µm filter.
- Ultrafiltration of sample (10 kDa MWCO) overnight in cold room.
- On ice, dissolve fatty acid free human serum albumin in the filtered sample, thereby obtaining a concentration of 10 mg HSA/ml.
- Add Na-caprylate in 5.4 times molar excess and place the sample in a water bath @ 65°C. When the temperature of the sample reaches 65°C, the incubation is continued for 45 minutes. Then the sample is allowed to cool to room temperature.
- Centrifugation @ 4000 × g, 5°C, 30 minutes, and filter the supernatant using a 0.45 µm filter.
- In a cold room, ultrafiltration of the sample (dilution using Milli-Q water) in order to remove excess Na-caprylate, pigments, etc. (10 kDa MWCO).
- Perform buffer-exchange to Dulbecco's Phosphate Buffered Saline + CaCl₂ + MgCl₂ (GIBCO^{™}, Ref. 14040-117) by diafiltration (10 kDa MWCO).
- Filtration using 0.22 µm filter.

The loaded HSA is now ready for use in serum-free culture medium for primary culture.

### Example 14. Use of loaded HSA compared to fatty acid free HSA

The cell line CHO-K1 was passaged in a growth medium containing nBSA (DMEM:F12 with Ultroser). The cells were then harvested, washed and plated at 500 cells/well in a growth medium without nBSA. Serial dilutions of rHSA obtained from expression in *Aspergillus oryzae,* and FAF nHSA (from Serologicals) was added to the cells and after 7 days of incubation, cell growth was measured as cell division by incorporation of a radioactively labeled nucleic acid analogue (³H-Thymidine). Results are given as counts per minute (cpm). The concentration of HSA in the medium is given in µg/ml. The rHSA obtained by expression in *A. oryzae* was treated as outlined below before use.

### Sample Preparation:

- Germ filtration
- Pasteurisation (adding 0.1 g Na-caprylate/L fermentation broth and heating to 65°C for 1 hour)
- Chromatographic capture step
- Pool fractions and store @ -18°C
- Thaw and filter using 0.22 µm filter
- Diafiltration using Milli-Q water and a 10 kDa MWCO filter
- Sterile filtration (0.22 µm filter)

The results are shown in figure 4 and indicates that HSA expressed in *Aspergillus oryzae* (rHSA) is superior to Fatty acid free HSA purified from human serum (FAF nHSA) with regards to the ability to stimulate cell growth. Cpm for cells cultured in the absence of SA was: 11.719. In a second experiment the cell line CHO-K1 was passaged in a growth medium containing nBSA (DMEM:F12 with Ultroser). The cells were then harvested, washed and plated at 10.000 cells/well in a growth medium without nBSA. Serial dilutions of "loaded" and "un-loaded" FAF nHSA (from Serologicals) was added to the cells and after 4 days of incubation, cell growth was measured as cell division, by incorporation of a radioactively labelled nucleic acid analogue (³H-Thymidine). Results are given as counts per minute (cpm). The concentration of HSA in the medium is given in µg/ml. Loading of FAF nHSA was performed essentially as outlined in example 13.

The results are shown in figure 5 and clearly indicate that loading of Fatty Acid Free (FAF) nHSA with albumin-free fermentation broth from *Aspergillus oryzae,* improves the ability of this nHSA to support cell growth. Cpm for cells cultured in the absence of SA was: 25.115.

### Example 15. Spray-drvinq of rHSA

A liquid composition consisting of 3 litre r-HSA solution containing about 97 g / litre was spray dried using a Two-Fluid-Nozzle (TFN) in a Mobile Minor spray dryer from the company Niro A/S, Denmark.

**Table 2: Drying conditions**

| **Batch no.** **(Lot. no)** | **Inlet temperature °C** | **Outlet temperature °C** | **Nozzle pressure Bar** | **Feed rate g/min.** |
|---|---|---|---|---|
| 1 | 120 | 55 | 3.5 | 6.5 |

The powder produced had the characteristics listed in Table 3.

**Table 3: Powder characteristics**

| **Bacth no.** **(Lot. no)** | **Bulk Density** **g/ml** | **Tapped Density** **g/ml** | **Angle of repose** **degrees** | **Particle Size** **D₅₀ µm** |
|---|---|---|---|---|
| 1 | 0.38 | 0.49 | 41 | 6 |

### Example 16. Spray-dryinq combined with an integrated fluid bed

A liquid feed preparation consisting of 57.7 kg r-HSA solution and 14.4 kg sodium chloride. The feed preparation had a dry matter content of about 28 %, where of r-HSA solids constituted ¼ of the total solids.

A Two-Fluid-Nozzle (TFN) was selected for the atomization of the feed. The spray dryer was equipped with an integrated fluid bed attached to the lower part of the conical bottom part of the drying chamber. The spent drying air was removed from the cylindrical drying chamber through the chamber roof. The air with entrained small particles were lead to a cyclone to separate of the entrained particle, so they could get reintroduced into the drying chamber through an annular slit surrounding the atomizing nozzle.

**Table 4: Drying conditions**

| **Batch no.** **(Lot. no)** | **Inlet temperature** **°C** | **Outlet temperature** **°C** | **Nozzle pressure** **Bar** | **Feed rate** **kg/g.** |
|---|---|---|---|---|
| 3 | 140 | Start: 75 Granulation: 67 | Start: 3.50 Granulation: 2.25 | 30 |

**Table 5: Powder characteristics**

| **Bacth no.** **(Lot. no)** | **Bulk Density** **g/ml** | **Tapped Density** **g/ml** | **Angle of repose** **degrees** | **Particle Size** **D₅₀ µm** |
|---|---|---|---|---|
| 3 | 0.37 | 0.43 | 34 | 133 |

The particle size was significantly increased due to the changed processing conditions of this example compared to the reference conditions in Example 15. The resulting product was in addition more free-flowing than the reference as the angle of repose was reduced. The bulk and tapped densities were, however, not significantly changed.

It is not simple to quantify if a powder is "easy" or "less easy" to re-constitute into a solvent. For many wide spread products this property is, however, of great importance. The International Dairy Federation (IDF), has developed a standard for measuring wettability, dispersibility and solubility (IDF Standard 087:1979 - Determination of the dispersibility & wettability). In Table 6 the results are summarized. The methods are based on IDF Standard # 087 and adopted to r-HSA. In all cases the same amount of active r-HSA was used.

**Table 6: Reconstitution properties of standard spray dried vs. new product**

| **Batch no.** **(Lot. no)** | **Wettability** **Min.** | **Dispersibility** **Sec.** | **Solubility** **%** |
|---|---|---|---|
| 1 | 8-10 | 120 | 75 |
| 3 | 3 | 50 | 93 |

Wettability: 2 g of new product and 0.5 g of standard product was added to the surface of 100 ml water (22 °C) in a 600 ml beaker. Time for complete dissolution was measured.

Dispersibility: 4 g of new product and 1 g of standard product was added to 100 ml of water (25 °C) in a 600 ml beaker during gentle manual stirring with a spoon. The time for complete dissolution was measured.

Solubility: 10 g of new product and 2.5 g of standard product was added to 100 ml water (25 °C) and stirred with a spoon for 20 sec. A sample of the liquid phase was taken and the dry matter content was measured. The percentage of solids in the solution was calculated.

The results found in Table 6 all show a remarkable improvement of the reconstitution properties of the new product according to this invention.

### Example 17. Spry-dried and agglomerated liquid feed product comprising HSA

A liquid feed preparation consisting of 100 kg HSA solution containing about 100 g/l HSA and 10 kg or 1 kg or 0.1 kg or 0.0 kg of a salt, preferable sodium chloride, is prepared. The feed preparation is added to a spray drying apparatus of the same set-up as disclosed in Example 16. A product with improved wettability, dispersibility and solubility is obtained when compared to a non-agglomerated dry HSA product.

## Claims

1. A dry particle which is agglomerated, wherein said particle comprises serum albumin.

2. The dry agglomerate of claim 1, wherein the agglomerate has a particle size above 50 microns.

3. A dry particle comprising serum albumin, wherein the particle has a particle size above 50 microns.

4. The dry particle of any of the claims 1-3, wherein the amount of serum albumin is above 50 wt% of the total agglomerate.

5. The dry particle of any of the claims 1-4, wherein the amount of serum albumin is above 75 wt% of the total agglomerate.

6. The dry particle of any of the claims 1-5, wherein the amount of serum albumin is above 90 wt% of the total agglomerate.

7. The dry particle of any of the claims 1-6, further comprising other ingredients.

8. The dry particle of claim 7, wherein the amount of other ingredients is less than 50 wt% of the total agglomerate.

9. A process for the preparation of the dry particle of any of the claims 1-8, comprising the steps of:
a) drying a liquid composition comprising serum albumin or recombinant serum albumin; and
b) agglomeration of the product of step a).

10. The process of claim 9, wherein the drying of the liquid composition is done by spray drying.

11. The process of claim 9, wherein the drying and agglomeration is performed in a fluidized spray dryer.

12. A process for the preparation of the particles of claim 3, comprising the steps of:
a) preparation of a liquid composition comprising serum albumin;
b) atomization of the liquid composition of step a); and
c) drying of the liquid composition to obtain dry particles with a particle size above 50 microns.

13. The process of claim 12, wherein the atomization is performed by use of a two fluid nozzle atomizer, a pressure nozzle atomizer or a rotary atomizer;

14. The process of claim 13, wherein the two fluid nozzle or the pressure nozzle atomizer is run at a pressure below 5 bar

15. The process of claim 13, wherein the rotary atomizer is run at a speed below 100 m/s.

16. The process of claims 9-15, further comprising a re-cycling step of primary particles.


SEQUENCE LISTING

<110>  Novozymes A/S

<120>  Recombinant production of serum albumin

<130>  10619

<160>  12

<170>  PatentIn version 3.2

<210>  1
<211>  1824
<212>  DNA
<213>  Bos taurus


<220>
<221>  sig_peptide
<222>  (1)..(54)

<220>
<221>  CDS
<222>  (1)..(1821)

<220>
<221>  misc_feature
<222>  (55)..(72)

<220>
<221>  mat_peptide
<222>  (73)..(1821)

<400>  1
atg aag tgg gtg act ttt att tct ctt ctc ctt ctc ttc agc tct gct      48
Met Lys Trp Val Thr Phe Ile Ser Leu Leu Leu Leu Phe Ser Ser Ala
                -20                 -15                 -10

tat tcc agg ggt gtg ttt cgt cga gat aca cac aag agt gag att gct      96
Tyr Ser Arg Gly Val Phe Arg Arg Asp Thr His Lys Ser Glu Ile Ala
            -5                  -1  1               5

cat cgg ttt aaa gat ttg gga gaa gaa cat ttt aaa ggc ctg gta ctg      144
His Arg Phe Lys Asp Leu Gly Glu Glu His Phe Lys Gly Leu Val Leu
    10                  15                  20

att gcc ttt tct cag tat ctc cag cag tgt cca ttt gat gag cat gta      192
Ile Ala Phe Ser Gln Tyr Leu Gln Gln Cys Pro Phe Asp Glu His Val
25                  30                  35                  40

aaa tta gtg aac gaa cta act gag ttt gca aaa aca tgt gtt gct gat      240
Lys Leu Val Asn Glu Leu Thr Glu Phe Ala Lys Thr Cys Val Ala Asp
                45                  50                  55

gag tcc cat gcc ggc tgt gaa aag tca ctt cac act ctc ttt gga gat      288
Glu Ser His Ala Gly Cys Glu Lys Ser Leu His Thr Leu Phe Gly Asp
            60                  65                  70

gaa ttg tgt aaa gtt gca tcc ctt cgt gaa acc tat ggt gac atg gct      336
Glu Leu Cys Lys Val Ala Ser Leu Arg Glu Thr Tyr Gly Asp Met Ala
        75                  80                  85

gac tgc tgt gag aaa caa gag cct gaa aga aat gaa tgc ttc ctg agc      384
Asp Cys Cys Glu Lys Gln Glu Pro Glu Arg Asn Glu Cys Phe Leu Ser
    90                  95                  100

cac aaa gat gat agc cca gac ctc cct aaa ttg aaa cca gac ccc aat      432

His Lys Asp Asp Ser Pro Asp Leu Pro Lys Leu Lys Pro Asp Pro Asn
105                 110                 115                 120

act ttg tgt gat gag ttt aag gca gat gaa aag aag ttt tgg gga aaa      480
Thr Leu Cys Asp Glu Phe Lys Ala Asp Glu Lys Lys Phe Trp Gly Lys
                125                 130                 135

tac cta tac gaa att gct aga aga cat ccc tac ttt tat gca cca gaa      528
Tyr Leu Tyr Glu Ile Ala Arg Arg His Pro Tyr Phe Tyr Ala Pro Glu
            140                 145                 150

ctc ctt tac tat gct aat aaa tat aat gga gtt ttt caa gaa tgc tgc      576
Leu Leu Tyr Tyr Ala Asn Lys Tyr Asn Gly Val Phe Gln Glu Cys Cys
        155                 160                 165

caa gct gaa gat aaa ggt gcc tgc ctg cta cca aag att gaa act atg      624
Gln Ala Glu Asp Lys Gly Ala Cys Leu Leu Pro Lys Ile Glu Thr Met
    170                 175                 180

aga gaa aaa gta ctg act tca tct gcc aga cag aga ctc agg tgt gcc      672
Arg Glu Lys Val Leu Thr Ser Ser Ala Arg Gln Arg Leu Arg Cys Ala
185                 190                 195                 200

agt att caa aaa ttt gga gaa aga gct tta aaa gca tgg tca gta gct      720
Ser Ile Gln Lys Phe Gly Glu Arg Ala Leu Lys Ala Trp Ser Val Ala
                205                 210                 215

cgc ctg agc cag aaa ttt ccc aag gct gag ttt gta gaa gtt acc aag      768
Arg Leu Ser Gln Lys Phe Pro Lys Ala Glu Phe Val Glu Val Thr Lys
            220                 225                 230

cta gtg aca gat ctc aca aaa gtc cac aag gaa tgc tgc cat ggt gac      816
Leu Val Thr Asp Leu Thr Lys Val His Lys Glu Cys Cys His Gly Asp
        235                 240                 245

cta ctt gaa tgc gca gat gac agg gca gat ctt gcc aag tac ata tgt      864
Leu Leu Glu Cys Ala Asp Asp Arg Ala Asp Leu Ala Lys Tyr Ile Cys
    250                 255                 260

gat aat caa gat aca atc tcc agt aaa ctg aag gaa tgc tgt gat aag      912
Asp Asn Gln Asp Thr Ile Ser Ser Lys Leu Lys Glu Cys Cys Asp Lys
265                 270                 275                 280

cct ttg ttg gaa aaa tcc cac tgc att gct gag gta gaa aaa gat gcc      960
Pro Leu Leu Glu Lys Ser His Cys Ile Ala Glu Val Glu Lys Asp Ala
                285                 290                 295

ata cct gaa aac ctg ccc cca tta act gct gac ttt gct gaa gat aag     1008
Ile Pro Glu Asn Leu Pro Pro Leu Thr Ala Asp Phe Ala Glu Asp Lys
            300                 305                 310

gat gtt tgc aaa aac tat cag gaa gca aaa gat gcc ttc ctg ggc tcg     1056
Asp Val Cys Lys Asn Tyr Gln Glu Ala Lys Asp Ala Phe Leu Gly Ser
        315                 320                 325

ttt ttg tat gaa tat tca aga agg cat cct gaa tat gct gtc tca gtg     1104
Phe Leu Tyr Glu Tyr Ser Arg Arg His Pro Glu Tyr Ala Val Ser Val
    330                 335                 340

cta ttg aga ctt gcc aag gaa tat gaa gcc aca ctg gag gaa tgc tgt     1152
Leu Leu Arg Leu Ala Lys Glu Tyr Glu Ala Thr Leu Glu Glu Cys Cys
345                 350                 355                 360

gcc aaa gat gat cca cat gca tgc tat tcc aca gtg ttt gac aaa ctt     1200
Ala Lys Asp Asp Pro His Ala Cys Tyr Ser Thr Val Phe Asp Lys Leu
                365                 370                 375

aag cat ctt gtg gat gag cct cag aat tta atc aaa caa aac tgt gac     1248

Lys His Leu Val Asp Glu Pro Gln Asn Leu Ile Lys Gln Asn Cys Asp
380 385 390

caa ttc gaa aaa ctt gga gag tat gga ttc caa aat gcg ctc ata gtt 1296
Gln Phe Glu Lys Leu Gly Glu Tyr Gly Phe Gln Asn Ala Leu Ile Val
395 400 405

cgt tac acc agg aaa gta ccc caa gtg tca act cca act ctc gtg gag 1344
Arg Tyr Thr Arg Lys Val Pro Gln Val Ser Thr Pro Thr Leu Val Glu
410 415 420

gtt tca aga agc cta gga aaa gtg ggt act agg tgt tgt aca aag ccg 1392
Val Ser Arg Ser Leu Gly Lys Val Gly Thr Arg Cys Cys Thr Lys Pro
425 430 435 440

gaa tca gaa aga atg tcc tgt act gaa gac tat ctg agc ttg atc ctg 1440
Glu Ser Glu Arg Met Ser Cys Thr Glu Asp Tyr Leu Ser Leu Ile Leu
445 450 455

aac cgg ttg tgc gtg ctg cat gag aag aca cca gtg agt gaa aaa gtc 1488
Asn Arg Leu Cys Val Leu His Glu Lys Thr Pro Val Ser Glu Lys Val
460 465 470

acc aag tgc tgc aca gag tca ttg gtg aac aga cgg cca tgt ttc tct 1536
Thr Lys Cys Cys Thr Glu Ser Leu Val Asn Arg Arg Pro Cys Phe Ser
475 480 485

gct ctg aca cct gat gaa aca tat gta ccc aaa gcc ttt gat gag aaa 1584
Ala Leu Thr Pro Asp Glu Thr Tyr Val Pro Lys Ala Phe Asp Glu Lys
490 495 500

ttg ttc acc ttc cat gca gat ata tgc aca ctt ccc gat act gag aaa 1632
Leu Phe Thr Phe His Ala Asp Ile Cys Thr Leu Pro Asp Thr Glu Lys
505 510 515 520

caa atc aag aaa caa act gca ctt gtt gag ctg ttg aaa cac aag ccc 1680
Gln Ile Lys Lys Gln Thr Ala Leu Val Glu Leu Leu Lys His Lys Pro
525 530 535

aag gca aca gag gaa caa ctg aaa acc gtc atg gag aat ttt gtg gct 1728
Lys Ala Thr Glu Glu Gln Leu Lys Thr Val Met Glu Asn Phe Val Ala
540 545 550

ttt gta gac aag tgc tgt gca gct gat gac aaa gaa gcc tgc ttt gct 1776
Phe Val Asp Lys Cys Cys Ala Ala Asp Asp Lys Glu Ala Cys Phe Ala
555 560 565

gtg gag ggt cca aaa ctt gtt gtt tca act caa aca gcc tta gcc taa 1824
Val Glu Gly Pro Lys Leu Val Val Ser Thr Gln Thr Ala Leu Ala
570 575 580

<210> 2
<211> 607
<212> PRT
<213> Bos taurus

<400> 2

Met Lys Trp Val Thr Phe Ile Ser Leu Leu Leu Leu Phe Ser Ser Ala
-20 -15 -10

Tyr Ser Arg Gly Val Phe Arg Arg Asp Thr His Lys Ser Glu Ile Ala
-5 -1 1 5

His Arg Phe Lys Asp Leu Gly Glu Glu His Phe Lys Gly Leu Val Leu

10                  15                  20

Ile Ala Phe Ser Gln Tyr Leu Gln Gln Cys Pro Phe Asp Glu His Val
25                  30                  35                  40

Lys Leu Val Asn Glu Leu Thr Glu Phe Ala Lys Thr Cys Val Ala Asp
                45                  50                  55

Glu Ser His Ala Gly Cys Glu Lys Ser Leu His Thr Leu Phe Gly Asp
            60                  65                  70

Glu Leu Cys Lys Val Ala Ser Leu Arg Glu Thr Tyr Gly Asp Met Ala
        75                  80                  85

Asp Cys Cys Glu Lys Gln Glu Pro Glu Arg Asn Glu Cys Phe Leu Ser
    90                  95                  100

His Lys Asp Asp Ser Pro Asp Leu Pro Lys Leu Lys Pro Asp Pro Asn
105                 110                 115                 120

Thr Leu Cys Asp Glu Phe Lys Ala Asp Glu Lys Lys Phe Trp Gly Lys
                125                 130                 135

Tyr Leu Tyr Glu Ile Ala Arg Arg His Pro Tyr Phe Tyr Ala Pro Glu
            140                 145                 150

Leu Leu Tyr Tyr Ala Asn Lys Tyr Asn Gly Val Phe Gln Glu Cys Cys
        155                 160                 165

Gln Ala Glu Asp Lys Gly Ala Cys Leu Leu Pro Lys Ile Glu Thr Met
    170                 175                 180

Arg Glu Lys Val Leu Thr Ser Ser Ala Arg Gln Arg Leu Arg Cys Ala
185                 190                 195                 200

Ser Ile Gln Lys Phe Gly Glu Arg Ala Leu Lys Ala Trp Ser Val Ala
                205                 210                 215

Arg Leu Ser Gln Lys Phe Pro Lys Ala Glu Phe Val Glu Val Thr Lys
            220                 225                 230

Leu Val Thr Asp Leu Thr Lys Val His Lys Glu Cys Cys His Gly Asp
        235                 240                 245

Leu Leu Glu Cys Ala Asp Asp Arg Ala Asp Leu Ala Lys Tyr Ile Cys
    250                 255                 260

Asp Asn Gln Asp Thr Ile Ser Ser Lys Leu Lys Glu Cys Cys Asp Lys
265                 270                 275                 280

Pro Leu Leu Glu Lys Ser His Cys Ile Ala Glu Val Glu Lys Asp Ala

285 290 295

Ile Pro Glu Asn Leu Pro Pro Leu Thr Ala Asp Phe Ala Glu Asp Lys
300 305 310

Asp Val Cys Lys Asn Tyr Gln Glu Ala Lys Asp Ala Phe Leu Gly Ser
315 320 325

Phe Leu Tyr Glu Tyr Ser Arg Arg His Pro Glu Tyr Ala Val Ser Val
330 335 340

Leu Leu Arg Leu Ala Lys Glu Tyr Glu Ala Thr Leu Glu Glu Cys Cys
345 350 355 360

Ala Lys Asp Asp Pro His Ala Cys Tyr Ser Thr Val Phe Asp Lys Leu
365 370 375

Lys His Leu Val Asp Glu Pro Gln Asn Leu Ile Lys Gln Asn Cys Asp
380 385 390

Gln Phe Glu Lys Leu Gly Glu Tyr Gly Phe Gln Asn Ala Leu Ile Val
395 400 405

Arg Tyr Thr Arg Lys Val Pro Gln Val Ser Thr Pro Thr Leu Val Glu
410 415 420

Val Ser Arg Ser Leu Gly Lys Val Gly Thr Arg Cys Cys Thr Lys Pro
425 430 435 440

Glu Ser Glu Arg Met Ser Cys Thr Glu Asp Tyr Leu Ser Leu Ile Leu
445 450 455

Asn Arg Leu Cys Val Leu His Glu Lys Thr Pro Val Ser Glu Lys Val
460 465 470

Thr Lys Cys Cys Thr Glu Ser Leu Val Asn Arg Arg Pro Cys Phe Ser
475 480 485

Ala Leu Thr Pro Asp Glu Thr Tyr Val Pro Lys Ala Phe Asp Glu Lys
490 495 500

Leu Phe Thr Phe His Ala Asp Ile Cys Thr Leu Pro Asp Thr Glu Lys
505 510 515 520

Gln Ile Lys Lys Gln Thr Ala Leu Val Glu Leu Leu Lys His Lys Pro
525 530 535

Lys Ala Thr Glu Glu Gln Leu Lys Thr Val Met Glu Asn Phe Val Ala
540 545 550

Phe Val Asp Lys Cys Cys Ala Ala Asp Asp Lys Glu Ala Cys Phe Ala

555                 560                 565

Val Glu Gly Pro Lys Leu Val Val Ser Thr Gln Thr Ala Leu Ala
    570                 575                 580

<210>  3
<211>  1830
<212>  DNA
<213>  Homo sapiens

<220>
<221>  CDS
<222>  (1)..(1827)

<220>
<221>  sig_peptide
<222>  (1)..(54)

<220>
<221>  misc_feature
<222>  (56)..(72)

<220>
<221>  mat_peptide
<222>  (73)..(1827)

<400>  3
atg aag tgg gta acc ttt att tcc ctt ctt ttt ctc ttt agc tcg gct      48
tat tcc agg ggt gtg ttt cgt cga gat gca cac aag agt gag gtt gct      96
cat cgg ttt aaa gat ttg gga gaa gaa aat ttc aaa gcc ttg gtg ttg      144
att gcc ttt gct cag tat ctt cag cag tgt cca ttt gaa gat cat gta      192
aaa tta gtg aat gaa gta act gaa ttt gca aaa aca tgt gtt gct gat      240
gag tca gct gaa aat tgt gac aaa tca ctt cat acc ctt ttt gga gac      288
aaa tta tgc aca gtt gca act ctt cgt gaa acc tat ggt gaa atg gct      336
gac tgc tgt gca aaa caa gaa cct gag aga aat gaa tgc ttc ttg caa      384
cac aaa gat gac aac cca aac ctc ccc cga ttg gtg aga cca gag gtt      432
gat gtg atg tgc act gct ttt cat gac aat gaa gag aca ttt ttg aaa      480
aaa tac tta tat gaa att gcc aga aga cat cct tac ttt tat gcc ccg 528
gaa ctc ctt ttc ttt gct aaa agg tat aaa gct gct ttt aca gaa tgt 576
tgc caa gct gct gat aaa gct gcc tgc ctg ttg cca aag ctc gat gaa 624
ctt cgg gat gaa ggg aag gct tcg tct gcc aaa cag aga ctc aag tgt 672
gcc agt ctc caa aaa ttt gga gaa aga gct ttc aaa gca tgg gca gta 720
gct cgc ctg agc cag aga ttt ccc aaa gct gag ttt gca gaa gtt tcc 768
aag tta gtg aca gat ctt acc aaa gtc cac acg gaa tgc tgc cat gga 816
gat ctg ctt gaa tgt gct gat gac agg gcg gac ctt gcc aag tat atc 864
tgt gaa aat caa gat tcg atc tcc agt aaa ctg aag gaa tgc tgt gaa 912
aaa cct ctg ttg gaa aaa tcc cac tgc att gcc gaa gtg gaa aat gat 960
gag atg cct gct gac ttg cct tca tta gct gct gat ttt gtt gaa agt 1008
aag gat gtt tgc aaa aac tat gct gag gca aag gat gtc ttc ctg ggc 1056
atg ttt ttg tat gaa tat gca aga agg cat cct gat tac tct gtc gtg 1104
ctg ctg ctg aga ctt gcc aag aca tat gaa acc act cta gag aag tgc 1152
tgt gcc gct gca gat cct cat gaa tgc tat gcc aaa gtg ttc gat gaa 1200
ttt aaa cct ctt gtg gaa gag cct cag aat tta atc aaa caa aat tgt 1248
gag ctt ttt gag cag ctt gga gag tac aaa ttc cag aat gcg cta tta 1296
gtt cgt tac acc aag aaa gta ccc caa gtg tca act cca act ctt gta     1344
gag gtc tca aga aac cta gga aaa gtg ggc agc aaa tgt tgt aaa cat     1392
cct gaa gca aaa aga atg ccc tgt gca gaa gac tat cta tcc gtg gtc     1440
ctg aac cag tta tgt gtg ttg cat gag aaa acg cca gta agt gac aga     1488
gtc acc aaa tgc tgc aca gaa tcc ttg gtg aac agg cga cca tgc ttt     1536
tca gct ctg gaa gtc gat gaa aca tac gtt ccc aaa gag ttt aat gct     1584
gaa aca ttc acc ttc cat gca gat ata tgc aca ctt tct gag aag gag     1632
aga caa atc aag aaa caa act gca ctt gtt gag ctc gtg aaa cac aag     1680
ccc aag gca aca aaa gag caa ctg aaa gct gtt atg gat gat ttc gca     1728
gct ttt gta gag aag tgc tgc aag gct gac gat aag gag acc tgc ttt     1776
gcc gag gag ggt aaa aaa ctt gtt gct gca agt caa gct gcc tta ggc     1824
tta taa                                                             1830

<210>  4
<211>  609
<212>  PRT
<213>  Homo sapiens

<400>  4

<210>  5
<211>  1821
<212>  DNA
<213>  Artificial

<220>
<223>  Synthetic CDS for BSA

<220>
<221>  sig_peptide
<222>  (1)..(54)

<220>
<221>  CDS
<222>  (1)..(1821)

<220>
<221>  misc_feature
<222>  (55)..(72)

<400>  5
atg aaa tgg gtg acc ttc ata tcc ttg ctg ttg ctg ttc agc agt gcc        48
Met Lys Trp Val Thr Phe Ile Ser Leu Leu Leu Leu Phe Ser Ser Ala
1               5                   10                  15

tac tct agg ggc gtg ttc agg agg gac aca cac aag agt gag att gca        96
Tyr Ser Arg Gly Val Phe Arg Arg Asp Thr His Lys Ser Glu Ile Ala
            20                  25                  30

cat cgg ttc aaa gac ttg gga gaa gag cat ttc aag ggc ttg gtc ctc       144
His Arg Phe Lys Asp Leu Gly Glu Glu His Phe Lys Gly Leu Val Leu
        35                  40                  45

att gca ttc tcc caa tat ctt cag cag tgc ccg ttc gat gag cac gtc       192
Ile Ala Phe Ser Gln Tyr Leu Gln Gln Cys Pro Phe Asp Glu His Val
    50                  55                  60

aag ttg gtg aat gaa ctc acc gag ttt gcg aag aca tgc gta gcg gac       240
Lys Leu Val Asn Glu Leu Thr Glu Phe Ala Lys Thr Cys Val Ala Asp
65                  70                  75                  80

gag tcc cat gct ggc tgt gag aag tcc cta cat acg ctg ttc ggc gac       288
Glu Ser His Ala Gly Cys Glu Lys Ser Leu His Thr Leu Phe Gly Asp
                85                  90                  95

gag ctt tgc aaa gtg gca tcg ctc cga gag acg tat gga gat atg gca       336
Glu Leu Cys Lys Val Ala Ser Leu Arg Glu Thr Tyr Gly Asp Met Ala
            100                 105                 110

gac tgt tgc gag aag cag gag cca gaa cgc aat gaa tgt ttc ttg tcc       384
Asp Cys Cys Glu Lys Gln Glu Pro Glu Arg Asn Glu Cys Phe Leu Ser
        115                 120                 125

cac aag gac gac agt ccc gat ttg ccc aag ctc aag ccc gac ccc aac       432
His Lys Asp Asp Ser Pro Asp Leu Pro Lys Leu Lys Pro Asp Pro Asn
    130                 135                 140

aca ctc tgc gac gag ttc aaa gcc gat gag aag aaa ttc tgg gga aag 480
Thr Leu Cys Asp Glu Phe Lys Ala Asp Glu Lys Lys Phe Trp Gly Lys
145                 150                 155                 160

tat ctc tat gag atc gcg agg cgc cat ccc tac ttc tac gca ccg gaa 528
Tyr Leu Tyr Glu Ile Ala Arg Arg His Pro Tyr Phe Tyr Ala Pro Glu
                165                 170                 175

ttg ttg tac tac gca aat aag tac aac gga gtg ttt cag gag tgt tgc 576
Leu Leu Tyr Tyr Ala Asn Lys Tyr Asn Gly Val Phe Gln Glu Cys Cys
            180                 185                 190

cag gcc gag gac aag gga gcc tgt ctc ctt ccc aaa ata gaa aca atg 624
Gln Ala Glu Asp Lys Gly Ala Cys Leu Leu Pro Lys Ile Glu Thr Met
        195                 200                 205

agg gag aag gtg ctt gcg tct agc gcc cgg cag cgc ctc cgc tgt gca 672
Arg Glu Lys Val Leu Ala Ser Ser Ala Arg Gln Arg Leu Arg Cys Ala
    210                 215                 220

tcg atc cag aag ttc ggt gag cga gcc ctc aaa gca tgg tcg gtt gcc 720
Ser Ile Gln Lys Phe Gly Glu Arg Ala Leu Lys Ala Trp Ser Val Ala
225                 230                 235                 240

agg cta tcc caa aag ttc ccc aaa gca gaa ttc gtc gag gtg aca aaa 768
Arg Leu Ser Gln Lys Phe Pro Lys Ala Glu Phe Val Glu Val Thr Lys
                245                 250                 255

ttg gtc acc gac ctc acg aag gtg cac aag gag tgt tgt cat ggc gac 816
Leu Val Thr Asp Leu Thr Lys Val His Lys Glu Cys Cys His Gly Asp
            260                 265                 270

ctt ttg gag tgt gcc gat gac cgc gca gat ctg gca aaa tac att tgt 864
Leu Leu Glu Cys Ala Asp Asp Arg Ala Asp Leu Ala Lys Tyr Ile Cys
        275                 280                 285

gat aac caa gac acc atc tcc agc aag ctc aag gag tgt tgt gac aag 912
Asp Asn Gln Asp Thr Ile Ser Ser Lys Leu Lys Glu Cys Cys Asp Lys
    290                 295                 300

ccg ctt ttg gag aag agt cac tgt ata gcc gag gtc gaa aaa gac gcg 960
Pro Leu Leu Glu Lys Ser His Cys Ile Ala Glu Val Glu Lys Asp Ala
305                 310                 315                 320

att ccg gaa aac ttg cct ccc ctc acg gcc gac ttc gca gag gat aaa 1008
Ile Pro Glu Asn Leu Pro Pro Leu Thr Ala Asp Phe Ala Glu Asp Lys
                325                 330                 335

gat gta tgc aag aac tac cag gag gca aag gat gct ttc ctc ggc tcg 1056
Asp Val Cys Lys Asn Tyr Gln Glu Ala Lys Asp Ala Phe Leu Gly Ser
            340                 345                 350

ttc ttg tat gaa tac agc agg cgc cac cca gaa tat gct gtt tca gtc 1104
Phe Leu Tyr Glu Tyr Ser Arg Arg His Pro Glu Tyr Ala Val Ser Val
        355                 360                 365

ctt ctt cgc cta gcg aaa gaa tac gaa gcc acc ctc gaa gaa tgt tgt 1152
Leu Leu Arg Leu Ala Lys Glu Tyr Glu Ala Thr Leu Glu Glu Cys Cys
    370                 375                 380

gcg aaa gac gac ccc cac gca tgc tac tcc act gtg ttc gat aag ctc 1200
Ala Lys Asp Asp Pro His Ala Cys Tyr Ser Thr Val Phe Asp Lys Leu
385                 390                 395                 400

aag cac ctc gtc gac gag cct caa aac ctt atc aag cag aac tgt gat 1248
Lys His Leu Val Asp Glu Pro Gln Asn Leu Ile Lys Gln Asn Cys Asp
                405                 410                 415

cag ttc gag aaa ttg gga gaa tat ggc ttt cag aac gcc ctc ata gtg     1296
Gln Phe Glu Lys Leu Gly Glu Tyr Gly Phe Gln Asn Ala Leu Ile Val
            420                 425                 430

cgc tac acc cgg aag gtc ccc caa gtc tcc aca cca acc ctg gtc gaa     1344
Arg Tyr Thr Arg Lys Val Pro Gln Val Ser Thr Pro Thr Leu Val Glu
        435                 440                 445

gta tcc cgg tcg ttg ggc aag gtc ggt act cgc tgt tgc acg aag ccc     1392
Val Ser Arg Ser Leu Gly Lys Val Gly Thr Arg Cys Cys Thr Lys Pro
    450                 455                 460

gag agt gag agg atg ccc tgt acc gaa gac tac ctc tcg ctc atc ctc     1440
Glu Ser Glu Arg Met Pro Cys Thr Glu Asp Tyr Leu Ser Leu Ile Leu
465                 470                 475                 480

aat agg ttg tgt gtg ctc cat gag aag act ccc gtg tcg gag aag gtg     1488
Asn Arg Leu Cys Val Leu His Glu Lys Thr Pro Val Ser Glu Lys Val
                485                 490                 495

acc aag tgt tgt acc gaa tcg ctg gtg aac cgc agg cca tgt ttc tcg     1536
Thr Lys Cys Cys Thr Glu Ser Leu Val Asn Arg Arg Pro Cys Phe Ser
            500                 505                 510

gcg ctc aca ccc gac gaa acg tat gtc ccg aaa gcc ttc gac gaa aaa     1584
Ala Leu Thr Pro Asp Glu Thr Tyr Val Pro Lys Ala Phe Asp Glu Lys
        515                 520                 525

ttg ttc acg ttc cat gcg gac att tgc acg cta ccg gat aca gaa aaa     1632
Leu Phe Thr Phe His Ala Asp Ile Cys Thr Leu Pro Asp Thr Glu Lys
    530                 535                 540

cag ata aag aag caa acc gcc ttg gtc gag ttg ctc aag cat aag ccc     1680
Gln Ile Lys Lys Gln Thr Ala Leu Val Glu Leu Leu Lys His Lys Pro
545                 550                 555                 560

aag gcg acc gag gaa cag ctc aag acg gtt atg gag aac ttc gtc gcc     1728
Lys Ala Thr Glu Glu Gln Leu Lys Thr Val Met Glu Asn Phe Val Ala
                565                 570                 575

ttc gtc gat aaa tgt tgt gca gcc gac gac aag gag gct tgt ttt gcc     1776
Phe Val Asp Lys Cys Cys Ala Ala Asp Asp Lys Glu Ala Cys Phe Ala
            580                 585                 590

gtc gag ggt cca aag ctc gtt gtc agc acc cag acc gcc ctg gcg         1821
Val Glu Gly Pro Lys Leu Val Val Ser Thr Gln Thr Ala Leu Ala
        595                 600                 605


<210>  6
<211>  607
<212>  PRT
<213>  Artificial

<220>
<223>  Synthetic CDS for BSA

<400>  6

Met Lys Trp Val Thr Phe Ile Ser Leu Leu Leu Leu Phe Ser Ser Ala
1               5                   10                  15


Tyr Ser Arg Gly Val Phe Arg Arg Asp Thr His Lys Ser Glu Ile Ala
            20                  25                  30


His Arg Phe Lys Asp Leu Gly Glu Glu His Phe Lys Gly Leu Val Leu

35                  40                  45

Ile Ala Phe Ser Gln Tyr Leu Gln Gln Cys Pro Phe Asp Glu His Val
    50                  55                  60

Lys Leu Val Asn Glu Leu Thr Glu Phe Ala Lys Thr Cys Val Ala Asp
65                  70                  75                  80

Glu Ser His Ala Gly Cys Glu Lys Ser Leu His Thr Leu Phe Gly Asp
                85                  90                  95

Glu Leu Cys Lys Val Ala Ser Leu Arg Glu Thr Tyr Gly Asp Met Ala
            100                 105                 110

Asp Cys Cys Glu Lys Gln Glu Pro Glu Arg Asn Glu Cys Phe Leu Ser
        115                 120                 125

His Lys Asp Asp Ser Pro Asp Leu Pro Lys Leu Lys Pro Asp Pro Asn
    130                 135                 140

Thr Leu Cys Asp Glu Phe Lys Ala Asp Glu Lys Lys Phe Trp Gly Lys
145                 150                 155                 160

Tyr Leu Tyr Glu Ile Ala Arg Arg His Pro Tyr Phe Tyr Ala Pro Glu
                165                 170                 175

Leu Leu Tyr Tyr Ala Asn Lys Tyr Asn Gly Val Phe Gln Glu Cys Cys
            180                 185                 190

Gln Ala Glu Asp Lys Gly Ala Cys Leu Leu Pro Lys Ile Glu Thr Met
        195                 200                 205

Arg Glu Lys Val Leu Ala Ser Ser Ala Arg Gln Arg Leu Arg Cys Ala
    210                 215                 220

Ser Ile Gln Lys Phe Gly Glu Arg Ala Leu Lys Ala Trp Ser Val Ala
225                 230                 235                 240

Arg Leu Ser Gln Lys Phe Pro Lys Ala Glu Phe Val Glu Val Thr Lys
                245                 250                 255

Leu Val Thr Asp Leu Thr Lys Val His Lys Glu Cys Cys His Gly Asp
            260                 265                 270

Leu Leu Glu Cys Ala Asp Asp Arg Ala Asp Leu Ala Lys Tyr Ile Cys
        275                 280                 285

Asp Asn Gln Asp Thr Ile Ser Ser Lys Leu Lys Glu Cys Cys Asp Lys
    290                 295                 300

Pro Leu Leu Glu Lys Ser His Cys Ile Ala Glu Val Glu Lys Asp Ala

305                 310                 315                 320

Ile Pro Glu Asn Leu Pro Pro Leu Thr Ala Asp Phe Ala Glu Asp Lys
                325                 330                 335

Asp Val Cys Lys Asn Tyr Gln Glu Ala Lys Asp Ala Phe Leu Gly Ser
            340                 345                 350

Phe Leu Tyr Glu Tyr Ser Arg Arg His Pro Glu Tyr Ala Val Ser Val
        355                 360                 365

Leu Leu Arg Leu Ala Lys Glu Tyr Glu Ala Thr Leu Glu Glu Cys Cys
    370                 375                 380

Ala Lys Asp Asp Pro His Ala Cys Tyr Ser Thr Val Phe Asp Lys Leu
385                 390                 395                 400

Lys His Leu Val Asp Glu Pro Gln Asn Leu Ile Lys Gln Asn Cys Asp
                405                 410                 415

Gln Phe Glu Lys Leu Gly Glu Tyr Gly Phe Gln Asn Ala Leu Ile Val
            420                 425                 430

Arg Tyr Thr Arg Lys Val Pro Gln Val Ser Thr Pro Thr Leu Val Glu
        435                 440                 445

Val Ser Arg Ser Leu Gly Lys Val Gly Thr Arg Cys Cys Thr Lys Pro
    450                 455                 460

Glu Ser Glu Arg Met Pro Cys Thr Glu Asp Tyr Leu Ser Leu Ile Leu
465                 470                 475                 480

Asn Arg Leu Cys Val Leu His Glu Lys Thr Pro Val Ser Glu Lys Val
                485                 490                 495

Thr Lys Cys Cys Thr Glu Ser Leu Val Asn Arg Arg Pro Cys Phe Ser
            500                 505                 510

Ala Leu Thr Pro Asp Glu Thr Tyr Val Pro Lys Ala Phe Asp Glu Lys
        515                 520                 525

Leu Phe Thr Phe His Ala Asp Ile Cys Thr Leu Pro Asp Thr Glu Lys
    530                 535                 540

Gln Ile Lys Lys Gln Thr Ala Leu Val Glu Leu Leu Lys His Lys Pro
545                 550                 555                 560

Lys Ala Thr Glu Glu Gln Leu Lys Thr Val Met Glu Asn Phe Val Ala
                565                 570                 575

Phe Val Asp Lys Cys Cys Ala Ala Asp Asp Lys Glu Ala Cys Phe Ala

580                 585                 590

Val Glu Gly Pro Lys Leu Val Val Ser Thr Gln Thr Ala Leu Ala
        595                 600                 605

<210>  7
<211>  1827
<212>  DNA
<213>  Artificial

<220>
<223>  Synthetic CDS for HSA

<220>
<221>  CDS
<222>  (1)..(1827)

<220>
<221>  sig_peptide
<222>  (1)..(54)

<220>
<221>  misc_feature
<222>  (55)..(72)

<220>
<221>  mat_peptide
<222>  (73)..(1827)

<400>  7
atg aaa tgg gtc acc ttc atc tcc ctc ctc ttc ctg ttt tct agc gca      48
tat tcg cga ggc gtg ttc agg cgt gat gcg cat aag tcc gaa gtg gca      96
cac cgt ttc aaa gat ctc ggg gag gag aac ttc aag gcc ttg gtg ctc      144
ata gca ttc gca cag tat ctt cag cag tgt cca ttc gaa gac cac gtt      192
aaa ctc gtt aac gag gtc aca gag ttc gcc aag acc tgt gta gcc gat      240
gag tcg gcg gaa aat tgt gat aag agt ctg cac acc ctc ttc gga gat      288
aag ctt tgt acg gtg gcg acg ctc agg gaa acc tat ggt gag atg gcc      336
gac tgc tgc gcg aag cag gag ccc gag cgt aac gag tgc ttc ttg cag      384
cac aag gat gac aac ccc aac ctg cct cga ctc gta agg cca gag gtc      432
gat gtc atg tgt acg gca ttc cat gac aac gaa gag acc ttc tta aaa     480
aag tac ctg tac gag atc gca cgc cgc cac cct tac ttc tat gca ccc     528
gag ctc cta ttc ttc gcc aag cga tac aag gca gcc ttc acc gag tgt     576
tgt caa gca gcc gac aag gcg gcg tgt cta tta ccg aaa ttg gat gag     624
ctc agg gat gag ggg aag gcg tca tcc gca aag cag agg ttg aag tgt     672
gcg tcc ttg cag aag ttc ggt gag cgc gca ttc aaa gcg tgg gca gtg     720
gca cgc ctc agc cag cgt ttc cca aag gct gaa ttc gca gaa gta tcg     768
aag ctc gtg acc gat ctc acc aaa gtg cac acc gag tgc tgt cat ggt     816
gat ctg ctc gag tgt gca gac gat agg gcc gat ctc gcc aaa tac att     864
tgt gaa aac cag gac tca atc tcg agt aag cta aaa gag tgt tgt gag     912
aaa ccg ctg ctg gag aaa tcc cat tgc ata gcc gag gtg gaa aac gac     960
gag atg ccg gcc gat ctt cca agc ctg gct gcc gat ttc gtg gaa tcc     1008
aag gat gtt tgt aag aac tac gcc gag gcg aag gac gtt ttt ctc gga     1056
atg ttt ctg tac gag tat gcc agg agg cat ccc gat tac agc gtg gta     1104
ctc ctc ctg cgg ctc gca aaa acg tac gaa aca aca ctc gaa aag tgc     1152
tgc gcg gcc gcc gac cct cat gag tgt tac gca aag gtg ttc gat gag     1200
ttc aag ccc ctt gta gag gag ccg cag aac ctc atc aag caa aac tgt     1248
gag ctt ttc gaa cag ttg ggc gaa tat aag ttc caa aac gca ctc ctt     1296
gtc agg tac act aag aaa gtt ccc cag gta tcg aca ccc act ctt gtc     1344
gag gtc agc cga aac cta ggg aaa gtt ggt tcc aaa tgt tgt aag cat     1392
ccc gag gct aaa cgc atg ccg tgt gca gag gac tac ttg tcg gtg gtc     1440
ctc aac caa ctg tgc gtc ctc cat gag aag aca cca gtg tcc gac cgt     1488
gtg acc aag tgt tgt aca gaa tcc ctc gtc aac cgg cgt cca tgc ttc     1536
agc gcc ctt gaa gtc gat gaa act tac gtt ccc aaa gag ttc aat gcc     1584
gaa act ttc aca ttc cac gcg gac att tgt act ctc tcg gaa aag gaa     1632
cga cag att aaa aaa cag aca gcc ttg gtc gag ctt gtc aaa cac aag     1680
ccc aag gcg act aaa gaa cag ctg aaa gca gta atg gat gat ttc gcc     1728
gcc ttc gtc gag aag tgt tgt aaa gcc gac gac aag gag acc tgt ttt     1776
gca gag gag ggc aag aag ctg gtg gct gcc tcg cag gcc gct ttg ggt     1824
ttg                                                                 1827


<210>  8
<211>  609
<212>  PRT
<213>  Artificial

<220>
<223>  Synthetic CDS for HSA

<400>  8

<210>  9
<211>  39
<212>  DNA
<213>  Artificial

<220>
<223>  Primer

<400>  9
atggacggat ccacaatgaa gtgggtaacc tttatttcc                              39

<210>  10
<211>  45
<212>  DNA
<213>  Artificial

<220>
<223>  Primer

<400>  10
atggacccgc ggctcgagtt ataagcctaa ggcagcttga cttgc                       45

<210>  11
<211>  40
<212>  DNA
<213>  Artificial

<220>
<223>  Primer

<400>  11
gactcgggat ccacaatgaa gtgggtgact tttatttctc                             40

<210>  12
<211>  42
<212>  DNA
<213>  Artificial

<220>
<223>  Primer

<400>  12
gactcgccgc ggctcgagtt aggctaaggc tgtttgagtt ga                          42